Europäisches Patentamt

European Patent Office

Office européen des brevets

(11) Veröffentlichungsnummer: **0 407 647 B1**

(12) **EUROPÄISCHE PATENTSCHRIFT**

(45) Veröffentlichungstag der Patentschrift: **06.10.93**

(51) Int. Cl.⁵: **G06F  15/20**

(21) Anmeldenummer: **89112971.0**

(22) Anmeldetag: **14.07.89**

(54) **Einrichtung zur Bewertung ausgewählter Signalanteile in physiologischen Messsignalen, insbesondere von Spätpotentialen in Elektrokardiogrammen.**

(43) Veröffentlichungstag der Anmeldung:
**16.01.91 Patentblatt  91/03**

(45) Bekanntmachung des Hinweises auf die Patenterteilung:
**06.10.93 Patentblatt  93/40**

(84) Benannte Vertragsstaaten:
**AT CH DE ES FR GB IT LI NL SE**

(56) Entgegenhaltungen:
**EP-A- 0 155 670**

**MEDICAL & BIOLOGICAL ENGINEERING & COMPUTING, Nr. 5, September 1987, Seiten 533-542, Stevenage, GB; YAMAGUCHI: "Simulation of nonstationary spectral analysis of turbulence in the aorta using a modified autoregressive or maximum entropy (AR/ME) method"**

**COMPUTERS IN CARDIOLOGY, 7.-10. Oktober 1986, Seiten 35-40, Boston, US; E.J. BERBARI et al.: "Methods for analyzing cardiac late potentials"**

**ICASSP'82, Paris, FR, 3.-5. Mai 1982, Band 2, Seiten 729-732; C.L. NIKIAS et al.: "A new robust 2-D spectral estimation method and its application in cardiac data analyisis"**

(73) Patentinhaber: **Haberl, Ralph, Dr.med.**
**Medizinische Klinik I**
**Klinikum Grosshadern**
**Marchioninistrasse 15**
**D-81377 München(DE)**

Patentinhaber: **Steinbeck, Gerhard, Prof.Dr.med.**
**Medizinische Klinik I**
**Klinikum Grosshadern**
**Marchioninistrasse 15**
**D-81377 München(DE)**

(72) Erfinder: **Haberl, Ralph, Dr. med.**
**Georgenstrasse 3a**
**D-8033 Planegg(DE)**
Erfinder: **Schels, Hans F., Dipl.-Phys.**
**Behamstrasse 21**
**D-8000 München 21(DE)**

(74) Vertreter: **TER MEER - MÜLLER - STEINMEISTER & PARTNER**
**Mauerkircherstrasse 45**
**D-81679 München (DE)**

**Beschreibung**

Die Erfindung betrifft eine Einrichtung zur rechnergestützten Bewertung ausgewählter Signalanteile in physiologischen Meßsignalen, insbesondere von Spätpotentialen in Elektrokardiogrammen (EKGs), welche nach Vorverstärkung und Impedanzwandlung über einen Hauptverstärker einer auf einen festen Spannungspegel normierten Feinverstärkung unterworfen und analog/digital gewandelt als Eingabedaten in einen Speicher eingelesen und durch den Rechner einer Frequenzanalyse unterzogen werden.

Patienten nach Herzinfarkt sind vom Auftreten bedrohlicher Herzrhythmusstörungen (Kammertachykardien) gefährdet. 5 bis 10 % der Patienten nach Herzinfarkt versterben innerhalb eines Jahres am plötzlichen Herztod aus rhythmogener Ursache. Bisherige Verfahren zur Identifizierung dieser Risikogruppe (Langzeit-EKG, Belastungs-EKG, Ruhe-EKG) sind auch bei Anwendung selektiver digitaler Filtertechnik, wie sie in "Computers in Cardiology", 7 - 10. Oktober 1986, S.35-40, Boston, USA, The Computer Society of the IEEE beschrieben ist, entweder zu wenig sensitiv oder zu wenig spezifisch. In den 70er Jahren wurden im Oberflächen-EKG während Sinusrhythmus am Ende des QRS-Komplexes winzige Signalschwankungen (Amplitude ca. 1 bis 5 $\mu$V) entdeckt, sogenannte **Spätpotentiale**, die bei durch Rhythmusstörung gefährdeten Patienten nach Herzinfarkt signifikant häufiger vorkommen als bei Patienten mit guter Prognose.

Der Nachweis dieser sehr kleinen Potentiale liegt an der Grenze des technisch Möglichen. Bisherige Verfahren verwenden die Signalmittelung über Summenbildung, um das Signal-Rausch-Verhältnis zu verbessern. Die bisher am meisten benutzte Methode nach SIMSON (Lit. 1; vgl. beigefügten Anhang 1) filtert die EKG-Signale bidirektional mit einem 25 Hz-Hochpaßfilter und vereint die drei Kanäle zu einer Vektorgröße, dem gefilterten QRS-Komplex. Nachteile dieser Methode sind, daß
- Patienten mit Schenkelblock ausgeschlossen werden müssen,
- die Definitionen von "abnormal" vom Rauschniveau abhängen,
- die Definitionen von verschiedenen Arbeitsgruppen nicht einheitlich gehandhabt werden,
- Einzelschläge nicht untersucht werden können, und
- eine räumliche Aussage über einen der drei Kanäle nicht möglich ist.

Außerdem kann
- die Hochpaßfilterung durch Filterüberschwinger Anlaß zu falschen Ergebnissen sein.

Diese Nachteile sind auch mit neueren Methoden zur digitalen Signalverbesserung, beispielsweise mit der oben erwähnten selektiven digitalen Filtertechnik nicht zu umgehen. Eine Signalfilterung jedweder Art bringt immer die Gefahr, daß neben den Störsignalen und Rauschen auch das Nutzsignal, in diesem Fall die Spätpotentiale, entfernt werden. Grundsätzlich davon abzugrenzen sind somit Methoden, die durch Spektralanalyse eine Differenzierung einzelner Signalkomponenten anstreben.

Als Forschungsergebnisse in den USA und der Bundesrepublik Deutschland sind neuere Methoden zur Erkennung von Spätpotentialen bekannt geworden (vgl. Lit. 2 und 3), die davon ausgehen, daß Spätpotentiale aufgrund ihres spezifischen Frequenzgehalts in der sonst sehr niederfrequenten ST-Strecke des EKGs durch Spektralanalyse erfaßt werden können. Als Alogrithmus wurde hierzu die Fourier-Transformation verwendet.

Obwohl damit einige Nachteile der bisher üblichen Zeitdarstellungs-Untersuchung vermieden werden können (vgl. Lit. 3), ist die Analyse mit Fourier-Transformation mit Problemen behaftet:

1. Die Frequenzauflösung kurzer Zeitsegmente ist sehr schlecht. Am EKG sind längere Datensegmente jedoch nicht anwendbar, da dann nicht interessierende EKG-Abschnitte das Frequenzspektrum störend beeinflussen würden.

2. Die Aussage über die zeitliche Lokalisation der Spätpotentiale im untersuchten Zeitsegment ist nicht möglich.

3. Fensterfunktionen sind zur Verminderung spektraler Leckeffekte erforderlich; dies reduziert weiter die Frequenzauflösung und kann Nutzsignale an den Segmentgrenzen abschwächen oder auslöschen.

4. Die Analyse von Spätpotentialen in einer ST-Strecke, die noch QRS-Anteile enthält, ist problematisch, da steile Flanken erhöhte spektrale Amplitudenanteile liefern und zu Frequenzverzerrungen führen.

Der Erfindung liegt damit die Aufgabe zugrunde, eine Einrichtung anzugeben, mit der sich bestimmte amplitudenmäßig oder hinsichtlich ihrer spektralen Leistungsdichte außerordentlich kleine Signalanteile physiologischer Meßsignale, wie Herzaktionssignale, insbesondere Spätpotentiale in Oberflächen-Elektrokardiogrammen, besonders störungsarm auswerten und aufzeichnen lassen und mit der die Beobachtung singulärer Vorgänge, wie beispielsweise bei EKGs eine Einzelschlaganalyse, möglich ist.

Erfindungsgemäß weist eine Einrichtung der eingangs genannten Gattung zur rechnergestützten Bewertung von physiologischen Meßsignalen die im kennzeichnenden Teil des Patentanspruchs 1 angegebenen Merkmale auf.

Vorteilhafte Ergänzungen und Weiterbildungen des Erfindungsgedankens sind in abhängigen Patentansprüchen angegeben und werden nachfolgend in Einzelheiten ergänzt und erläutert.

Die Erfindung beruht auf der technischen Anwendung und besonderen Anpassung autoregressiver mathematischer Modelle zur Berechnung des Frequenzspektrums. Diese mathematischen Modelle ermöglichen eine Analyse mit der sogenannten "Maximum-Entropie-Methode",nachfolgend auch abgekürzt als MEM bezeichnet, und/oder mittels einer Analyse mit adaptiver Filterbestimmung, einer erfindungsbezogenen Weiterentwicklung der sogenannten "Fast Adaptive Forward/Backward Least-Squares-Methode", nachfolgend auch abgekürzt als AFB bezeichnet. Die eingesetzten Algorithmen wurden für die Auswertung von EKGs besonders angepaßt, wobei insbesondere neue Methoden zur Berechnung der optimalen Ordnung des autoregressiven Modells und Methoden zur Elimination störender niederfrequenter Grundschwingungen entworfen wurden.

Zur weiteren Erhöhung der Aussagekraft wird, soweit es sich um EKGs handelt, nicht nur ein Segment der ST-Strecke, sondern es wird eine Vielzahl von zeitlich in der ST-Strecke gegeneinander versetzter Segmente untersucht, beispielsweise 38 zeitlich um 2 ms innerhalb der ST-Strecke gegeneinander verschobener Segmente. In einer dreidimensionalen Darstellung - Amplitude gegen Frequenz gegen Zeit - läßt sich dann eine hohe Aussagekraft gewinnen. Aus diesen Einzelspektren wird dann ein "Normalitätsfaktor" berechnet, der angibt, ob in dem EKG des jeweiligen Patienten Spätpotentiale vorhanden sind oder nicht.

Die besonderen Vorteile, die sich mit der erfindungsgemäßen Einrichtung gewinnen lassen, sind:

a) Sehr hohe Frequenzauflösung auch bei kurzen Segmenten;

b) Verzicht auf Fensterfunktionen;

c) gute Abgrenzbarkeit von Spätpotentialen gegenüber Rauschen und Störeinflüssen;

d) eindeutige Definition von abnormen Spektren; die Definition ist nicht vom Rauschniveau abhängig;

e) durch die mögliche Verwendung von kurzen Zeitsegmenten sind Spätpotentiale innerhalb der ST-Strecke genau lokalisierbar.

Die Einrichtung vereinigt somit die Vorteile der Frequenzanalyse mit denen der Zeitdarstellung, jedoch mit dem wesentlichen Unterschied zu bisher bekannten Methoden, daß keine Hochpaßfilterung erforderlich ist, eine gute Abgrenzung gegenüber Störeinflüssen sowie eine exakte Definition von "Abnormal" möglich ist, ebenso wie eine eindeutige Lokalisierbarkeit signifikanter Signalanteile, bei EKGs z. B. der Spätpotentiale in der ST-Strecke.

Obwohl die Erfindung in erster Linie in Anwendung auf die Erfassung von Spätpotentialen in Oberflächen-EKGs beschrieben wird, die über drei bipolare Elektrodenpaare gewonnen werden, ist der Erfindungsgedanke nicht auf diese spezielle, derzeit jedoch bevorzugte Anwendung beschränkt. Mit der erfindungsgemäßen Einrichtung können auch andere biologische Signale hinsichtlich spezieller charakteristischer Signalanteile untersucht werden, beispielsweise EEGs, Herzschallkurven und dergleichen. Auch besteht keine prinzipielle Einschränkung hinsichtlich der Art der Signalableitung. So können beispielsweise anstelle von drei orthogonalen Elektrodenpaaren auch Ableitungen von Brustwandelektroden als Eingangssignale zugrunde gelegt werden, ebenso wie intrakardiale EKGs. Für die frequenzmäßige Auswertung der Spektren ist innerhalb vernünftiger physiologisch vorkommender Grenzwerte kein spezieller Bereich festgelegt, obgleich hinsichtlich der im Vordergrund stehenden Spätpotentiale in erster Linie Spektren im Bereich von 40 bis 160, eventuell bis 200 Hz, in Frage kommen.

Ein besonders interessantes Anwendungsgebiet der Erfindung besteht in der Analyse des QRS-Komplexes zur Abstoßungsdiagnostik bei Herztransplantationen sowie bei der Analyse von Segmenten vor Beginn des QRS-Komplexes, insbesondere zur Erfassung des sogenannten His-Potentials. Das His-Potential entsteht im His'schen Bündel an der Vorhof-Kammergrenze und ist im konventionellen EKG wegen zu geringer Amplitude nicht erkennbar. Im Frequenzspektrum zeigt der HIS-Spike erhöhtem Frequenzinhalt.

Bei Patienten nach Herztransplantation zeigt die Frequenzanalyse des QRS-Komplexes eine Frequenzzunahme im Bereich 60 bis 150 Hz und eine Frequenzabnahme der ST-Strecke im Bereich 10 bis 50 Hz während akuter Abstoßungsreaktionen. Gegenüber der bisher angewandten Fourier-Transformation erlaubt die erfindungsgemäße Frequenzanalyse mit autoregressivem Algorithmus der MEM bzw. AFB eine hochgenaue Erfassung und Lokalisierung dieser Frequenzschwankungen im EKG.

Die Erfindung und vorteilhafte Einzelheiten werden nachfolgend unter Bezugnahme auf die Zeichnung näher erläutert. Es zeigen:

Fig. 1    den Blockschaltbildaufbau einer erfindungsgemäßen Einrichtung zur Erkennung und Bewertung von Spätpotentialen in Oberflächen-EKGs;

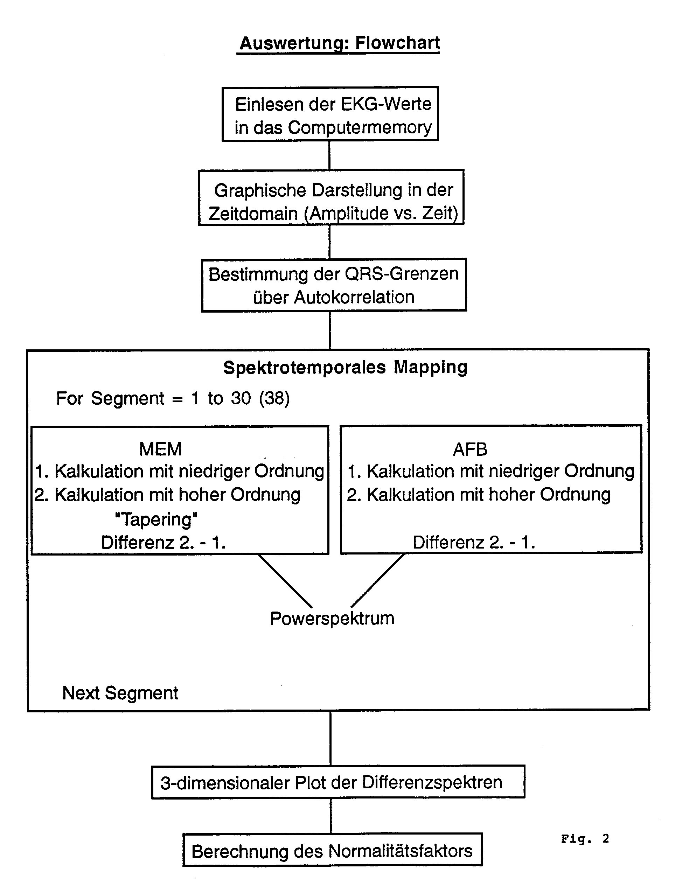

Fig. 2    ein Flußdiagramm zur Erläuterung der einzelnen Schritte der Signalauswertung;

Fig. 3    einen einzelnen QRS-Komplex, anhand dessen die Arbeitsweise des Verfahrens und der erfindungsgemäßen Einrichtung erläutert wird;

Fig. 4    drei Diagramme, die charakteristische Spätpotentiale für einen Patienten nach einem Herzinfarkt bei Anwendung der beiden alternativen Auswertungsmethoden gemäß der Erfindung veranschaulichen;

Fig. 5    drei der Darstellung nach Fig. 4 entsprechende Diagramme für einen Herzgesunden;

Fig. 6    drei der Darstellung nach Fig. 4 entsprechende Signaldiagramme zur Erläuterung, wie sich mit der erfindungsgemäßen Einrichtung Störeinflüsse von Spätpotentialen eindeutig unterscheiden lassen; und

Fig. 7    zum Vergleich mit der Erfindung Amplitude/Frequenz/Zeit-Diagramme von ST-Streckenausschnitten, die gemäß herkömmlichen Verfahren einer Fourier-Analyse unterzogen wurden.

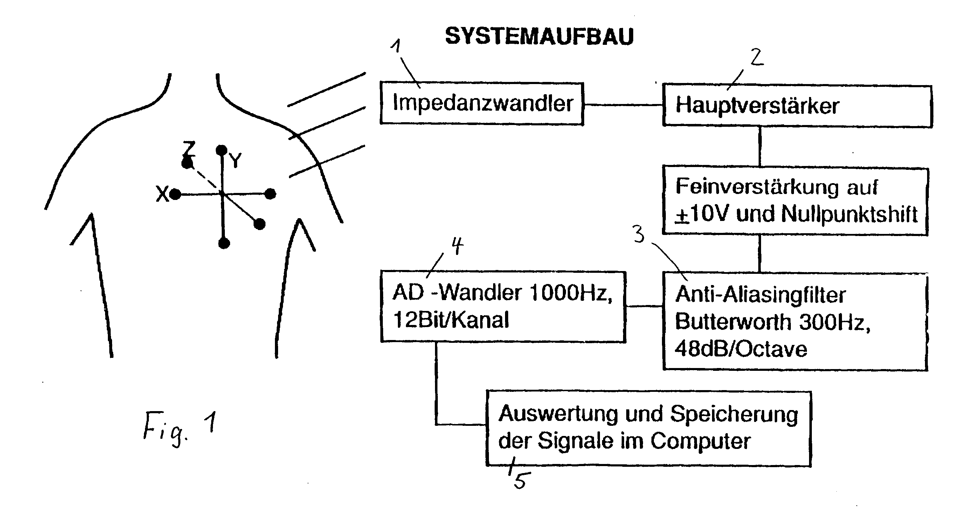

Der Systemaufbau einer erfindungsgemäßen Einrichtung wird zunächst unter Bezug auf die Fig. 1 erläutert:

Von drei bipolaren Elektrodenpaaren X, Y, Z werden orthogonale Elektrokardiogramme abgeleitet. Ein Vorverstärker 1 mit Impedanzwandlung in unmittelbarer Nähe der Elektrodenpaare minimiert die Störeinstrahlung aus der Umgebung. In einem Hauptverstärker 2 werden die EKG-Signale auf ± 10 V verstärkt, wobei rechnergesteuert in Abhängigkeit von der Amplitude des Eingangssignals eine Feinregulierung der Verstärkung und eine künstliche DC-Verschiebung des Signals erfolgt, was die volle Nutzung des Eingangsbereichs eines A/D-Wandlers (± 10 V) erlaubt. Der A/D-Wandler 4 arbeitet im erprobten Ausführungsbeispiel der Erfindung mit 12 Bit Auflösung und 1000 Hz Abtastrate. Die simultan aufgenommenen EKG-Signale werden auf einer Festplatte gespeichert und in einem Computer verarbeitet. Während der Entwicklungsarbeiten und zahlreicher Tests hat sich ein technischer 32 Bit Rechner der Firma Hewlett Packard, Serie 9000 mit einer Taktrate von 16 MHz sehr bewährt. Die Analyse und Auswertung der gespeicherten Daten geschieht wie folgt:

Die Rohdaten des EKGs liegen beispielsweise als 16 Bit Integer-Werte für einen Bereich von $\pm 2^{12}$ ($\pm 2048$) vor. Zunächst werden die Rohdaten mit dem Verstärkungsfaktor verrechnet und vorzugsweise als Real-Zahlen in Mikrovolt angegeben. Beginn und Ende des QRS-Komplexes werden über Autokorrelation berechnet. Dazu wird zunächst die räumliche Vektorgeschwindigkeit des EKGs mit Hilfe der Formel

$$v(n) = \sqrt{(x(n+1)-x(n))^2 + (y(n+1)-y(n))^2 + z(n+1)-z(n))^2}/dt$$

berechnet, worin mit

$v(n)$    die räumliche Vektorgeschwindigkeit,

$n$    die Datenpunkte $n = 1, ..., N, N+1$

$dt$    die Abtastrate und mit

$x, y, z$    die von den Elektrodenpaaren X, Y, Z gelieferten Signale bezeichnet sind.

Nach einer groben Abschätzung des QRS-Endes, beispielsweise so, daß der Punkt, bei dem die Vektorgeschwindigkeit 20 mV/s unterschreitet, durchläuft ein QRS-Signalsegment von beispielsweise 80 ms Zeitdauer, beginnend bei einem Punkt "abgeschätztes QRS-Ende minus 20 ms" den mathematischen Algorithmus der Autokorrelation über Faltung. Im Ergebnis erhält man eine zeitliche Reihe. Der Zeitpunkt des Faltungsmaximums definiert das endgültige QRS-Ende als Bezugspunkt für die weitere Auswertung. Mit demselben Vorgehen wird der QRS-Beginn bestimmt.

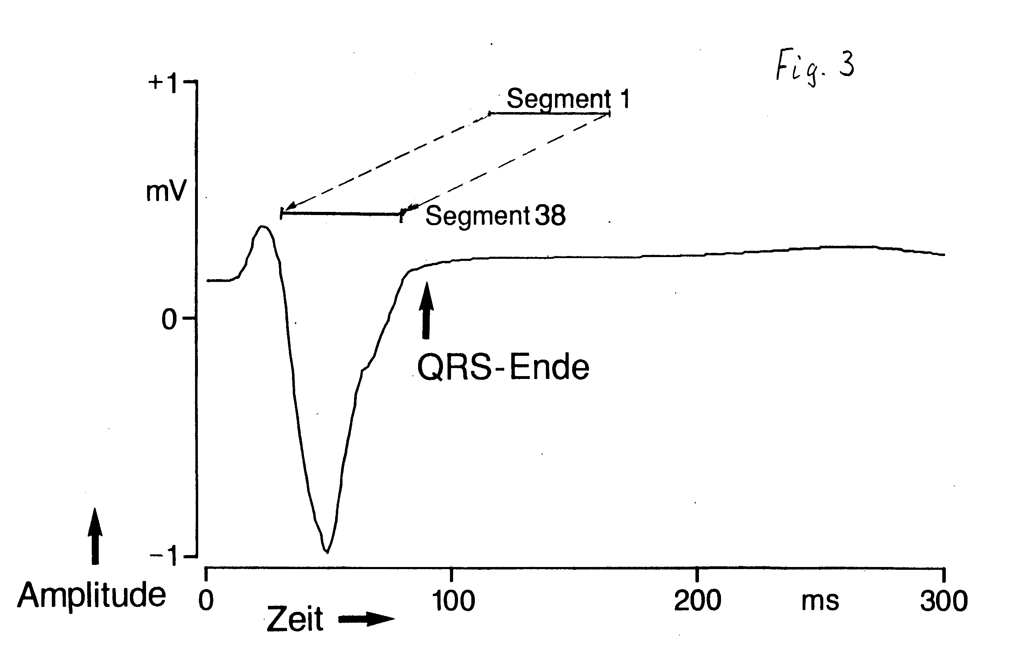

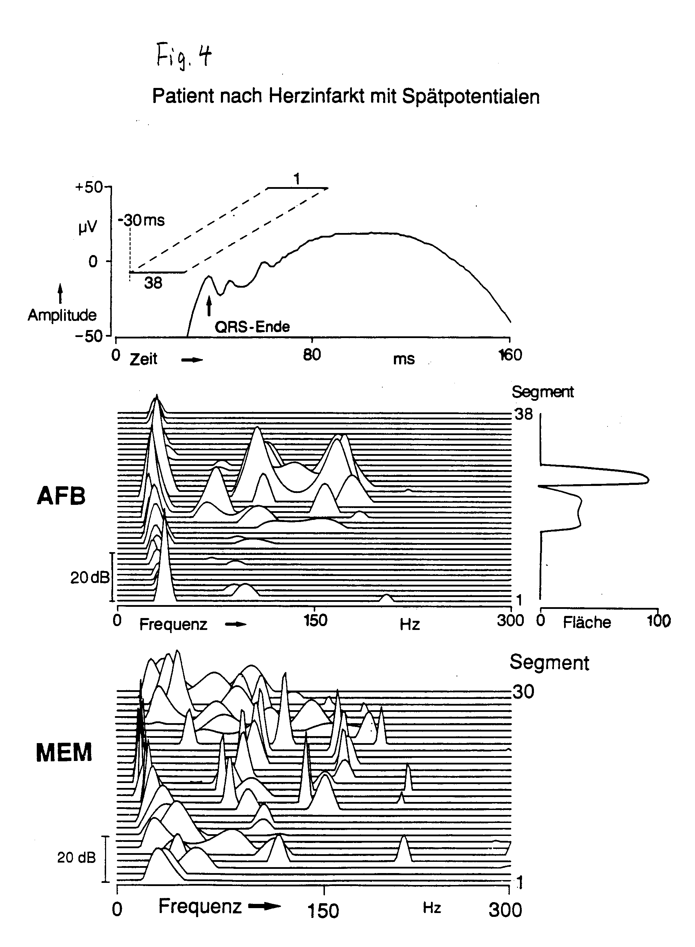

Nun wird eine bestimmte Anzahl von Zeitsegmenten, beispielsweise 38 Segmente, innerhalb der ST-Strecke definiert. Wie die Fig. 3 und Fig. 4 (oben) erkennen lassen, beginnt das erste Segment beispielsweise 44 ms nach dem Ende des QRS-Komplexes; die nachfolgenden beginnen jeweils 2 ms früher im Herzzyklus. Das 38. Segment beginnt somit 30 ms innerhalb des QRS-Komplexes. Für MEM-Auswertung (siehe unten) beträgt die Segmentlänge beispielsweise 40 ms, während man bei adaptiver Filterbestimmung mit der AFB-Methode (siehe unten) sogar mit kürzeren Segmentlängen von beispielsweise 25 ms ausgekommt. Von jedem dieser Segmente wird das Leistungsspektrum berechnet.

Die in der erfindungsgemäßen Einrichtung bewirkte Berechnungs- und Auswertemethode wurde von den Erfindern als "Spektrotemporales Mapping" bezeichnet und wird so als Fachbegriff eingeführt werden.

Die beiden für die technische Lösung des aufgezeigten Problems eingesetzten Berechnungsmethoden werden nachfolgend kurz beschrieben.


**Analyse mittels Maximum Entropie Methode (MEM)**

Die Berechnung der spektralen Leistungsdichte einer Zeitreihe über die Berechnung eines autoregressiven Modells (AR-Modell) bestimmter Ordnung ist auf der von Burg (1978) beschriebenen Methode begründet (Lit. 4). Die Verringerung der Prädiktionsfehlerfunktion erfolgt über die Minimierung der Summe der quadrierten Vorwärts- und Rückwärtsfehler. Gleichzeitig muß bei der rekursiven Berechnung der Parameter der sogenannte Levinson-Durbin-Algorithmus erfüllt sein. Das Spektrum der MEM läßt sich geschlossen darstellen durch die Gleichung

$$MEM(M,n) = \sigma^2 \cdot \delta t / \left[ \sum_{m=1}^{M} \cdot a(M,m) \cdot \exp\{-2 \cdot \pi \cdot i \cdot (m-1) \cdot n \cdot \delta t\} \right]^2 \qquad (1)$$

mit

$\sigma^2$:       Spektrale Leistungsdichte des weißen Rauschens;

N:       Anzahl der verfügbaren Daten, n = 1, ..., N;

M:       Modellordnung, m = 1, ..., M;

$\delta t$:       Abtastrate

a(M,m):       Koeffizienten des Prädiktions-Fehlerfilters mit der Länge M und a(M,1) = 1.

Die vorwärts- bzw. rückwärtsgerichteten Prädiktionsfehler (Fehler der Kovarianzfunktion) einer Zeitreihe x(n) der Länge N zur Zeit n sind gegeben durch v(M,n) und r(M,n). Dabei stellt M die aktuelle Ordnung der Rekursion über die AR-Parameter dar. Es gilt:

$$v(M,n) = x(n) + \sum_{m=1}^{M} a(M,m) \cdot x(n-m) \qquad (2)$$

und

$$r(M,n) = x(n-m) + \sum_{m=1}^{M} a(M,m) \cdot x(n-M+m) \qquad (3)$$

a(M,1) = 1, n = 1, ... N, m = 1, ..., M

v(M,n), r(M,n) sind nicht-korrelierte Signalfolgen (weißes Rauschen) mit dem Erwartungswert Null und der Varianz $\sigma^2$, welche als mittlerer quadratischer Prädiktionsfehler interpretiert werden kann.

Die Berechnung beider Fehler erfolgt über dieselben Koeffizienten; das Signal verläuft jedoch in entgegengesetzten Richtungen. Dies wird im folgenden kurz erläutert:

Die vorliegenden Daten x(n) sind der Ausgang bzw. die Antwortwortgröße des unbekannten linearen Systems S(z), ein Filter, mit z = exp[2·π·i·f], mit f = Frequenz. Über dieses unbekannte Filter S(z) wird aus eintreffendem weißem Rauschen das aufzuzeichnende Signal x(n) erzeugt.

Das Spektrum von x(n) entspricht der quadrierten Filterfrequenzantwort und es gilt:

$$F(f) = |S(z)|^2 \cdot s(x)^2$$

mit

$|S(z)|^2$:       Filterleistungsantwort,

$s(x)^2$:       Leistung des weißen Rauschens am Beginn von S(z),

F(f):       spektrale Leistungsdichte des Signals x(n),

f:       Frequenz,

z:       exp[2·π·i·f].

Läßt sich aus weißem Rauschen durch das Filter S(z) die Funktion x(n) bilden, so muß aus der Funktion x(n) durch das zu S(z) inverse Filter $S^n(z)$ mit n = -1 wiederum weißes Rauschen zu erzeugen sein.

Die Funktion S(z) mit

$$S(z) = 1/(1 - \sum_{m=1}^{M-1} a(M,m)/z^n)$$

5

wird als All-Pol-Übertragungsfunktion bezeichnet. Die dazu inverse Funktion ist $S^n(z)$, $n = -1$, mit

$$S^n(z) = 1 - \sum_{m=1}^{M-1} a(M,m)/z^n$$

wird als FIR- oder All-Zero-Übertragungsfunktion bezeichnet.

Die Koeffizienten $a(M,m)$ werden nun so bestimmt, daß die Leistung des weißen Rauschens am Ausgang minimiert wird, wenn das Signal $x(n)$ über das inverse Filter läuft. Dies geschieht, indem die Daten, respektive in der weiteren Rekursion die Fehlerfunktion vorwärts und rückwärts durch das Filter geschickt und ihre jeweiligen Energien, d. h. die Summe über die Vorwärts- und Rückwärtsfehler, $v(M,n)$ und $r(M,n)$ verringert werden. Der erfindungsgemäß eingesetzte Algorithmus macht sich hier die Eigenschaft linearer Filtersysteme zunutze, daß ein Filter, welches sowohl auf das vorwärts als auch auf das rückwärts gerichtete Signal angewendet wird, dasselbe Ergebnis liefert. Die Leistung von Vorwärts- und Rückwärtsfehlerfunktion entspricht der Leistung des weißen Rauschens.

Zur Berechnung der AR-Parameter wird die Summe der Energien der vorwärts und rückwärts gerichteten Fehlerfunktion minimiert entsprechend der Gleichung

$$\sigma^2(M) = \sum_{n=M+1}^{N} \{v(M,n)^2 + r(M,n)^2\} \qquad (4)$$

unter der Bedingung, daß die AR-Parameter die Levinson/Durbin-Rekursion

$$a(M,m) = a(M-1,m) + a(M,M) \cdot a(M-1,M-m) \qquad (5)$$

für alle Ordnungen $m$ von 1 bis $M-1$ erfüllen. Diese Forderung dient der Erzeugung eines stabilen AR-Filters (Pole innerhalb des Einheitskreises). Die Minimierung wird dadurch erreicht, daß die Ableitung der Fehlerterme nach $a(M,m)$ zu Null gesetzt wird.

Daraus folgt als Ausdruck für die AR-Parameter $a(M,M)$ der M-ten Ordnung:

$$a(M,M) = - \frac{2 \cdot \sum_{n=M+1}^{N} \{v(M-1,n) \cdot r(M-1,n-1)\}}{\sum_{n=m+1}^{N} \{v(M-1,n)^2 + r(M-1,n-1)^2\}} \qquad (6)$$

Die Energie des gesamten Fehlers respektive die Varianz oder die Dynamik des Fehlersignals folgt zu:

$$P(M) = P(M-1) \cdot [1-a(M,M)^2] \qquad (7)$$

Der schematisierte Ablauf des MEM-Algorithmus nach Burg läßt sich zusammengefaßt in folgenden Schritten darstellen:
-   Initialisierung:

$$\sigma^2 = \sum_{i=1}^{N} x(i)^2,$$

Ordnung = 1
-   Beginn mit der Ordnung = 1,
    -- Berechnung der AR-Parameter (Gl. (6))
        -- Minimierung der Fehlerfunktion unter Beachtung der Levinson/Durbin-Rekursion (Gl. (4), (5))

-- Prädiktionsfehlerbestimmung (Gl. (3), (2)),
- Erhöhung der Ordnung und erneuter Durchlauf, beginnend mit Berechnung der AR-Parameter sodann
- Berechnung des Spektrums (Gl. (1)).

Ein wesentlicher Punkt, den es beim Einsatz der MEM zur Frequenzanalyse des EKGs zu beachten gilt, ist die Wahl der richtigen Ordnung des autoregressiven Modells, die repräsentiert wird durch die Anzahl der AR-Parameter respektive der Prädiktionsfilterkoeffizienten. Im Rahmen der Entwicklungsarbeiten wurde festgestellt, daß bei einer zu niedrig gewählten Ordnung das Spektrum sehr stark geglättet und die Frequenzauflösung herabgesetzt wird. Die Wahl einer zu hohen Ordnung hingegen führt zu zusätzlichen, im Originalsignal nicht existierenden Anteilen im Spektrum.

Bekannte Selektionsverfahren zur Bestimmung einer optimalen Ordnung erbrachten keine voll befriedigenden Ergebnisse, da keines der bekannten Verfahren alle Anforderungen in bezug auf spektrale Auflösung, gewünschte Segmentlänge, tolerierbare Rechenzeit usw. erfüllten.

Als sehr vorteilhafte Ergänzung und Verbesserung des Erfindungsgedankens wurde eine Verfahrensführung entwickelt, bei der eine optimale Festlegung der Ordnung als Abbruchkriterium für die Rekursion erreicht wurde. Gemäß dieser Ergänzung der Erfindung wird das Abbruchkriterium aus der Kombination dreier unterschiedlicher Selektionskriterien bestimmt, bei denen die Modellordnung in der Regel relativ hoch angesetzt wird [7]. Dasjenige Kriterium, das als erstes die Abbdruchbedingung erfüllt, legt den Grad der Rekursion fest.

Für das erste dieser drei Kriterien lautet die Abbruchbedingung FPE (FPE = $\underline{F}$inal $\underline{P}$rediction $\underline{E}$rror Criterion):

$$\{FPE(m) = \sigma^2 \bullet (n+m)/(n-m)\} > \{FPE(m-1)\},$$

mit m = 1, ..., M.

Darin bezeichnet M die Modellordnung; für n gilt n = 1, ..., N; N bezeichnet die Anzahl der Daten. Demgemäß muß der Wert eines neuen Fehlerkriteriums für einen beliebigen Wert m größer sein als sein Vorgänger um die Abbrechbedingung zu erfüllen.

Das nächste Kriterium für einen Rekursionsabbruch ist die Bedingung für ein stabiles Filter: Das Ende der Rekursion ist erreicht, wenn der berechnete Filterkoeffizient größer als 1 wird, d. h.:

Stop der Rekursion, wenn: a(M,m) > 1.

Mögliche numerische Ungenauigkeiten, welche zu Fehlern führen können, werden auf diese Weise vermieden.

Sollte keines dieser beiden Kriterien den Abbruch herbeiführen, so wird die Rekursion nach Erreichen einer vorgegebenen oberen Grenze für die Anzahl der Koeffizienten des Prädiktions-Fehlerfilters beendet. Diese obere Grenze wird in Abhängigkeit von der Anzahl der Daten innerhalb eines fest vorgegebenen Datensegments berechnet; sie wird beispielsweise bestimmt zu einem Drittel der Anzahl der Datenmenge der Wurzel der aktuellen Segmentlänge. Damit folgt für die späteste Beendigung der Rekursion:

Ende der Rekursion bei: m > 3 $\bullet \sqrt{N}$

Wird bei MEM zur Analyse des EKGs die Ordnung bei der Bestimmung der genannten Koeffizienten zu hoch gewählt, so kann dies zum Auftreten von Line-Splitting und Frequenzverschiebungen führen. Unter Line-Splitting wird das Aufspalten eines Frequenzgipfels in zwei oder mehrere Gipfel verstanden, welche um die eigentliche Frequenz lokalisiert sind Die Frequenzverschiebung, d. h. die Abweichung einer Frequenz von ihrem ursprünglichen Wert, kann bis zu 16 % der jeweils betrachteten sogenannten Frequenzauflösungszelle betragen Beide Phänomene können außerdem vom Startpunkt, also von der Phasenlage und der Kurvenform der jeweils betrachteten Segmente abhängig sein.

Gemäß einer weiteren vorteilhaften Ergänzung der Erfindung lassen sich beide Probleme weitgehend beseitigen durch Anwendung einer Taperfunktion auf die Berechnung der Filterkoeffizienten nach Gleichung (6) [8].

Die als vorteilhaft gefundene Taperfunktion hat die Form:

$$T(m,n) = 6 \bullet (m+1) \bullet (N-n-m+1)/[(N-n+1) \bullet (N-n+2) \bullet (N-n+3)]$$

worin
m = 1, ..., M; M: Modell-Ordnung und
n = 1, ..., N; N: Anzahl der Daten
bezeichnen.

Überraschenderweise hat sich gezeigt, daß bei Erweiterung der Gleichungsbeziehung für die Filterkoeffizienten (Gleichung (6)) im Zähler und Nenner um die Taperfunktion auch für höhere Werte der Modellordnung M Line-Splitting und Frequenzverschiebungen nicht mehr auftraten.

Gemäß der Erfindung werden vor allem bisher beobachtete störende Beeinträchtigungen der EKG-Analyse beseitigt, die darauf beruhen, daß niederfrequente Grundschwingungen, verursacht in erster Linie durch die Grundschwingung der S-Zacke des QRS-Komplexes bzw. der aszendierenden ST-Strecke, niederfrequente Anteile in den Spektren hervorrufen, die weit in den Frequenzbereich f ≧ 40 Hz hineinwirken können, in dem Spätpotentiale auftreten Eine naheliegende Lösung wäre eine Hochpaßfilterung der Ausgangsdaten; dies verursacht jedoch, wie oben erwähnt, bestimmte Nachteile, insbesondere Signalverzerrungen.

Mit der Erfindung wurden die Nachteile herkömmlicher Filterung überwunden und die erwähnte Grundschwingung wird nach folgendem Verfahren eliminiert:

Die Grundschwingung wird zusammen mit einem entsprechenden Rauschuntergrund durch die oben erwähnte Signalanalyse bei sehr niedriger Modellordnungszahl, z. B. 1, 2 oder 3, erfaßt. Anschließend wird derselbe Abschnitt mit der optimalen Ordnung untersucht, für die das Rekursionsabbruchkriterium erfüllt ist. Eine Subtraktion des Spektrums niedriger Modellordnung vom Spektrum mit optimaler Modellordnung auf der Ebene der Berechnung der Filterkoeffizienten liefert schließlich ein Spektrum mit einem sehr niedrigen Rauschniveau, in welchem die Grundschwingung praktisch vollständig reduziert ist.

Bei gleichzeitiger Anwendung der beiden genannten Verbesserungen des Erfindungsgedankens auf die Berechnung der Differenzspektren, nämlich bei Berechnung der Filterkoeffizienten mit optimaler Modellordnung und bei Anwendung der Taper-Modifikation lassen sich Leistungsspektren gewinnen, die im Vergleich zu herkömmlichen Methoden mit Fourier-Analyse eine wesentlich bessere Aussagekraft ergeben.

## Analyse mittels AFB-Methode

Eine andere, im Rahmen der Erfindung wahlweise anwendbare Methode zur Bestimmung des EKG-Leistungsspektrums verwendet in vorteilhafter Ausgestaltung der Erfindung einen Algorithmus, dem die als "Fast Adaptive Forward-Backward Least Squares" bekannt gewordene Methode zugrunde liegt - ein autoregressives Verfahren, das zum ersten Mal 1987 vorgestellt wurde von N. Kalouptsidis und S. Theodoridis (Lit. 6). In der erfindungsgemäß modifizierten Form läßt sich diese Methode auch als "Adaptive Filterbestimmung" (AFB) bezeichnen.

Das Konzept dieser Methode beruht auf der schrittweisen Anpassung der Filterparameter an die Eingangsfunktion unter simultaner Minimierung der Energien der vorwärts- und Rückwärts-Prädiktionsfehler. Im Gegensatz zur oben beschriebenen Maximum-Entropie-Methode (MEM) erfolgt die Modellierung des Eingangssignals schrittweise von Datenpunkt zu Datenpunkt bei fest vorgegebener Anzahl der Filterkoeffizienten (feste Modellordnung M).

Das Leistungsdichtespektrum der AFB-Methode ist analog zu Gleichung (1) bei MEM gegeben durch:

$$AFB(M,n) = \sigma^2 \delta t / \left[ \sum_{m=1}^{M} a(M,m) \cdot \exp\{-2 \cdot \pi \cdot i \cdot (m-1) \cdot n \cdot \delta t\} \right]^2$$

$\sigma^2$:       Spektrale Leistungsdichte des weißen Rauschens;

N:       Anzahl der verfügbaren Daten, n = 1, ..., N;

M:       Modell-Ordnung, m = 1, ..., M;

$\delta t$:       Abtastrate

a(M,m):       Filterkoeffizienten

Die vorwärts bzw. rückwärts gerichteten Prädiktionsfehler einer Zeitreihe x(n) der Länge N zur Zeit n sind, ähnlich wie oben bereits erläutert, gegeben durch v(M,n) und r(M,n). Dabei stellt M wiederum die Anzahl der Filterparameter respektive die Höhe der Modell-Ordnung dar. Es gilt:

$$v(M,n) = \dot{x}(n) + \sum_{m=1}^{M} J \cdot a(M,m) \cdot x(n-m) \qquad (8)$$

und

$$r(M,n) = x(n-m) + \sum_{m=1}^{M} a(M,m) \cdot x(n-M+m) \qquad (9)$$

v(M,n), r(M,n) sind nicht-korrelierte Signalfolgen (weißes Rauschen) mit dem Erwartungswert Null und der Varianz $\sigma^2$, welche wiederum als mittlerer quadratischer Prädiktionsfehler interpretiert werden kann.

Das Filter J•a(M,m) wird als Vorwärts-Prädiktor bezeichnet mit J als sogenannter Austauschmatrix, die definiert ist als:

$$J \;=\; J(M) \;=\; \begin{bmatrix} 0 & \dots & 0 & \dots & 1 \\ \cdot & & & & \cdot \\ \cdot & & & & \cdot \\ 0 & \dots & 1 & \dots & 0 \\ \cdot & & & & \cdot \\ \cdot & & & & \cdot \\ 1 & \dots & 0 & \dots & 0 \end{bmatrix}$$

Das Filter a(M,m) wird als Rückwärts-Prädiktor bezeichnet.

Die Minimierung der Summe der Energien der vorwärts und rückwärts gerichteten Fehlerfunktion bzw. Filterfunktion liefert:

$$\sigma^2(M) \;\approx\; \sum_{n=M+1}^{N} \left\{ v(M,n)^2 + r(M,n)^2 \right\} \qquad (10)$$

Der AFB-Algorithmus macht keine Annahme über das Signal außerhalb des betrachteten bzw. vorliegenden Datenbereichs. Die Energien von Vorwärts- und Rückwärts-Prädiktionsfehler werden simultan minimiert.

Ein großer Vorteil des AFB-Algorithmus ist seine Unabhängigkeit von der Startphase des Signals, so daß Line-Splitting nicht zu beobachten ist. Als weiterer Vorteil ergibt sich eine hohe Frequenzauflösung bei kaum beobachteter Frequenzverschiebung auch bei sehr kurzen Datensegmenten.

Zur Erzielung optimaler Ergebnisse im Rahmen der Erfindung ist es auch bei Anwendung des AFB-Algorithmus wichtig, die Anzahl der verwendeten Filterparameter richtig zu wählen, d. h. die optimale Ordnung zu bestimmen. Diese ist abhängig von den vorliegenden Daten und läßt sich nur schwer im voraus festlegen. Trotz bekanntem AFB-Algorithmus sind Methoden zur Bestimmung der optimalen Ordnung nicht bekannt. Eine zu niedrig gewählte Ordnung glättet das Spektrum zu sehr und kann eine Minderung der Frequenzauflösung bedeuten. Die Wahl einer zu hohen Ordnung andererseits führt zu zusätzlichen, im Originalsignal nicht vorhandenen Anteilen im Spektrum.

Als vorteilhafte Ergänzung des Erfindungsgedankens hat sich folgendes Selektionskriterium zur Bestimmung der optimalen Ordnung besonders bewährt:

Der Algorithmus wird mit einer festen oberen Schranke (= Anzahl der Punkte in einem Signalabschnitt oder Segment), maximal jedoch 50, gestartet. Nach jedem Rekursionsschritt wird geprüft, ob die jeweils letzten Filterfehler (Ausgang vorwärts/rückwärts) eine bestimmte Bedingung erfüllen, d. h. innerhalb eines vorgegebenen Bereichs liegen. Liegen die Fehler außerhalb dieses Bereichs, so wird die Rekursion abgebrochen; der aktuelle Laufindex der Rekursion wird gespeichert. Mit diesem aktuellen Laufindex wird die Rekursion anschließend neu gestartet und die Filterkoeffizienten werden berechnet. Zur Überprüfung der Effizienz und der Gültigkeit der jeweils gefundenen Ordnung wird ein Verfahren zur Beurteilung der Stabilität des jetzt vorliegenden Filters bestimmt durch die Filterkoeffizienten eingesetzt. Dieses Überprüfungsverfahren ist bekannt und von B. Friedlander ausführlich beschrieben (Lit. 5).

Eine zweite Möglichkeit zur Festlegung einer optimalen Ordnung ergibt sich mit einem Kriterium, wie es oben in Verbindung mit MEM bereits beschrieben wurde, wonach als obere Schranke für ein Ende der Rekursion $m > 3\sqrt{N}$ festgelegt ist.

Die Berechnung der Differenzspektren erfolgt aufgrund ähnlicher Überlegungen, wie oben bei MEM bereits beschrieben.

Die erzielten Ergebnisse zeigen, daß bei der AFB-Methode die Verwendung noch kürzerer Segmente möglich ist, z. B. Segmentlängen von nur 25 ms, was die Lokalisation von Spätpotentialen in der ST-Strecke wesentlich verbessert.

Ein weiterer Vorteil der AFB-Methode liegt darin, daß artifizielle Frequenzberge weniger häufig auftreten und die Methode daher, wie die nachfolgenden Ausführungen zeigen, in manchen Anwendungsfällen noch spezifischer ist als MEM.

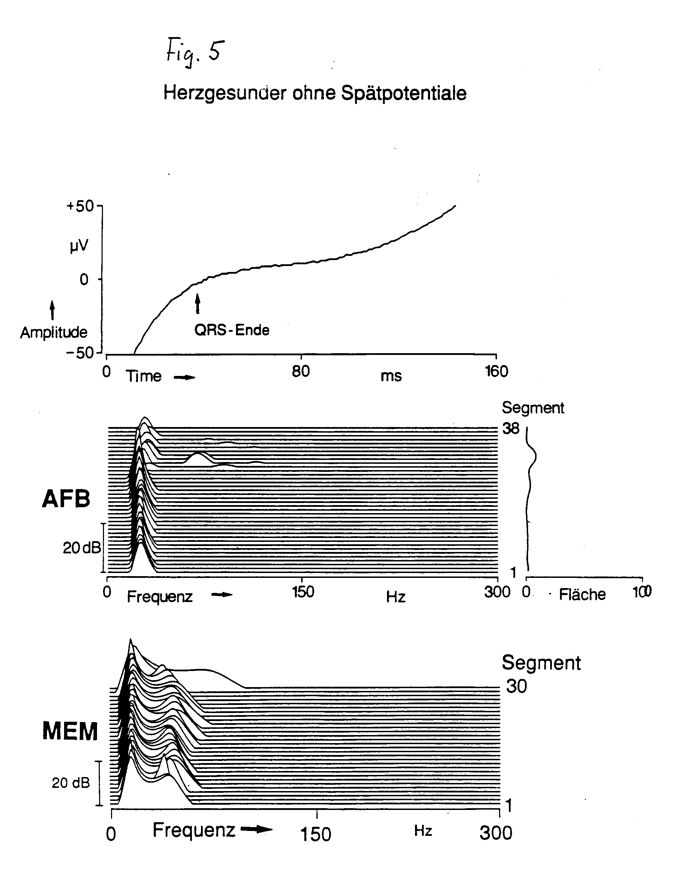

Wie die Darstellungen der Fig. 4 bis 7 erkennen lassen, werden die Leistungsspektren in einer dreidimensionalen Darstellung graphisch aufgetragen, wobei der Blickwinkel beliebig gewählt werden kann. Je ein pathologisches und ein normales Beispiel ist für MEM und AFB in den Fig. 4 bzw. 5 dargestellt.

Der erste Patient (Fig. 4) erlitt einen Herzinfarkt mit nachfolgend anhaltenden Kammertachykardien in der Anamnese.

Der zweite Patient (Fig. 5) hatte ebenfalls einen Herzinfarkt erlitten, jedoch ergaben sich keine Hinweise auf eine anhaltende ventrikuläre Rhythmusstörung.

In beiden Fällen der Fig. 4 bzw. 5 zeigt sich sowohl bei der adaptiven Frequenzbestimmung (jeweils mittleres Bild AFB) als auch bei der Maximum-Entropie-Methode (jeweils unteres Bild MEM) ein Frequenzgipfel bei niederer Frequenz (ca. 15 Hz), der durch alle Segmente 1 bis 38 (AFB) bzw. 1 bis 30 (MEM) hindurchgeht und der niederfrequenten Grundschwingung der ST-Strecke entspricht. Aufgrund der Differenzbildung der Spektren mit hoher und niederer Modell-Ordnung M stört dieser Frequenzgipfel die Analyse nicht.

Im Bereich über 50 Hz treten beim Patienten gemäß Fig. 4 höherfrequente Anteile auf, die vor allem in Segmenten am QRS-Ende (vgl. oberes Diagramm der Fig. 4), d. h. in den Segmenten 19 bis 27 (mittleres Diagramme der Fig. 4, AFB) bzw. in den Segmenten 12 bis ca. 23 (unteres Diagramm in Fig. 4, MEM) lokalisiert sind, jedoch in Segmenten weit außerhalb des QRS-Komplexes, d. h. in den Segmenten 1 bis 18 (AFB) bzw. 1 bis 13 (MEM) fehlen. Dies ist ein charakteristischer Befund für Spätpotentiale, der sich aus dem mittleren Diagramm der Fig. 4 (AFB) noch deutlicher ablesen läßt. Das Spektraldiagramm rechts von AFB zeigt deutlich die aufsummierte Leistungsverteilung mit unübersehbarer Leistungsspitze im Frequenzbereich der Spätpotentiale. Der Normalitätsfaktor, der weiter unten noch näher erläutert wird, ist pathologisch. Aufgrund der kurzen Segmentlänge bei AFB sind die Spektren mit Frequenzbergen (entsprechender Spätpotentiale) eindeutig abgrenzbar und bei AFB noch deutlicher lokalisierbar. Damit ist ein exakter Rückschluß auf die Lokalisation der Spätpotentiale in der Zeitdarstellung (oberes Diagramm der Fig. 4 und 5) möglich.

Beim Patienten ohne Kammertachykardien gemäß Fig. 5 fehlen die hochfrequenten Anteile in den Segmenten am QRS-Ende; damit besteht kein Hinweis auf das Vorhandensein von Spätpotentialen.

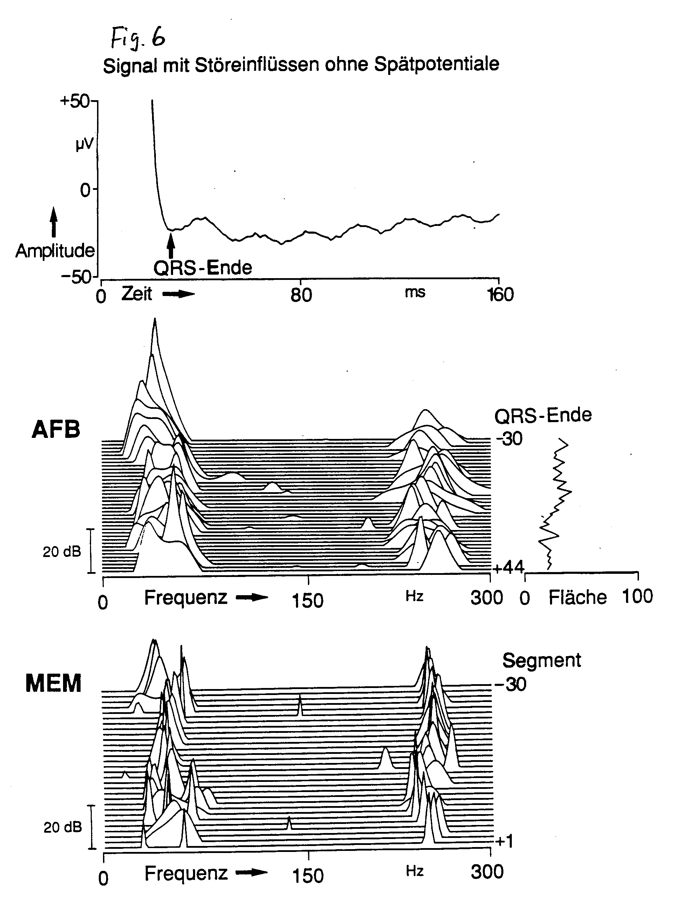

Fig. 6 zeigt zun Unterschied der Fig. 4 und 5 die Analyse eines EKGs mit der erfindungsgemäßen Einrichtung bei vorhandenen Störeinflüssen. Diese führen ebenfalls zu hochfrequenten Anteilen, die jedoch in allen Segmenten mehr oder weniger einheitlich vorhanden sind. Bei der nachfolgend erläuterten Bestimmung des Normalitätsfaktors fallen diese Störeinflüsse jedoch heraus.

Die **Berechnung des Normalitätsfaktors** geht von der Vorstellung aus, daß Spätpotentiale nur in Segmenten um das QRS-Ende vorhanden sind, jedoch nicht in Segmenten weit außerhalb des QRS-Komplexes vorkommen. Störeinflüsse, im wesentlichen verursacht durch Elektrodenrauschen, Muskelzittern und Netzeinstreuung sollten dagegen in allen Spektren mehr oder weniger gleichmäßig auftreten (vgl. als Anschauungsbeispiel Fig. 6). Aus früheren Untersuchungen mit Fourier-Transformation (vgl. Lit. 3) ist es bekannt, daß Spätpotentiale in allererster Linie nur im Frequenzbereich von 40 Hz bis 200 Hz vertreten sind.

Mit der erfindungsgemäßen Einrichtung wird in einem **ersten Schritt** zunächst geprüft, ob im Frequenzbereich 40 bis 160 Hz in den Spektren bestimmter Segmente, insbesondere der Segmente 14 bis 30, Frequenzgipfel mit einer Amplitude > 6 dB vorkommen. Ist dies nicht der Fall, so ist dieser Befund mit Spätpotentialen nicht vereinbar; der Normalitätsfaktor ist 100 %. Treten entsprechende Gipfel jedoch auf, so folgt der nächste Schritt.

Im **Schritt zwei** wird durch den Rechner geprüft, ob ein im ersten Schritt gefundener, relevanter Frequenzgipfel nur in den ausgewählten Segmenten (z. B. 14 bis 30) vorliegt oder ob ein solcher Gipfel in einem Frequenzband von ± 10 Hz der entsprechenden Frequenz in den Spektren aller Segmente auftaucht. Ist dies der Fall, so ist von der spektralen Repräsentation eines Störsignals auszugehen und nicht von Spätpotentialen. Im Programmablauf wird jetzt zurückgegangen auf Schritt 1 und es wird geprüft, ob ein weiterer relevanter Frequenzgipfel vorhanden ist. Ist jedoch ein relevanter Frequenzgipfel lediglich in den Segmenten um das QRS-Ende lokalisiert und weit außerhalb des QRS-Komplexes nicht vorhanden, so folgt jetzt der dritte Schritt.

Im **dritten Schritt** wird die spektrale Ausprägung der Spätpotentiale erfaßt. Dazu wird das Flächenintegral unter der Frequenzkurve (vgl. mittleres und unteres Schaubild der Fig. 4 bis 6) im Bereich 40 bis 160 Hz in den Spektren der zuvor ausgewählten Segmente, also insbesondere der Segmente 14 bis 30 einerseits, und in den übrigen Spektren, also insbesondere den Spektren 1 bis 13 andererseits, berechnet.

Der Normalitätsfaktor NF ist definiert als Quotient, nämlich

$$NF = \frac{(\text{Flächenintegral der Spektren 1 bis 13})}{(\text{Flächenintegral der Spektren 14 bis 30})}$$

Signifikante Spätpotentiale sind bei einem Normalitätsfaktor NF < 0,3 anzunehmen.

Für die exakte Lokalisierung der Spätpotentiale wird das Flächenintegral im Bereich von 40 bis 160 Hz als Funktion aller Segmentnummern, beispielsweise der Segmente 1 bis 38, aufgetragen; vgl. Schaubild rechts vom jeweils mittleren Bild - AFB - der Fig. 4 bis 6. Der Beginn von Spätpotentialen wird festgelegt als das Segment, bei dem das Flächenintegral einen bestimmten Wert übersteigt. Das Ende der Spätpotentiale wird entsprechend bestimmt als dasjenige Segment, dessen spektrale Fläche unter diesen Grenzwert fällt.

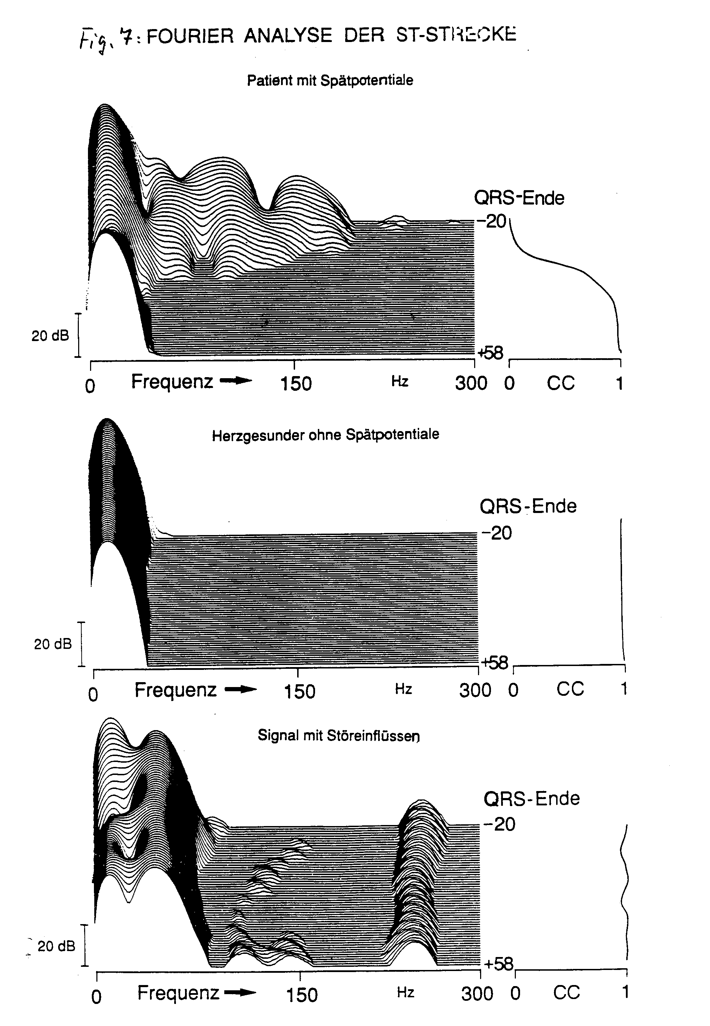

Die drei Diagramme der Fig. 7, welche zum Vergleich mit Fourier-Analyse der ST-Strecke in bekannter Weise, jedoch mit analoger segmentweiser Betrachtung aufgenommen wurden, bedürfen für den Fachmann keiner weiteren Erläuterung. Das obere Schaubild der Fig. 7, das für den gleichen Patienten mit Spätpotentialen wie bei Fig. 4 aufgenommen wurde, läßt deutlich erkennen, wie viel ungenauer die Lokalisierungsmöglichkeit für Spätpotentiale ausfällt. Das mittlere Diagramm der Fig. 7 wäre mit der Darstellung der Fig. 5 und das untere Diagramm der Fig. 7 mit den Diagrammen der Fig. 6 zu vergleichen.

Es folgen:

Anhang 1:  Literaturliste
Anhang 2:  Programmausschnitte zur Berechnung der Filterkoeffizienten nach MEM
Anhang 3:  Programmausschnitte zur Berechnung der Filterkoeffizienten nach AFB

## Anhang 1

LITERATUR:

1. Simson MB., Use of Signals in the Termal QRS-Complex to Identify Patients with Ventricular Tachycardia after Myocardial Infarction. Circulation 1981; 64: 235

2. Cain ME., Amboss HD., Witkowsky FX., Sobel BE., Fast Fourier Transform Analysis of Signal Averaged Electro-cardiograms for Identification of Patients Prone to Sustained Ventricular Tachycardia. Circulation 1984; 69: 711

3. Haberl R., Jilge G., Pulter R., Steinbeck G., Comparison of Frequency and Time Domain Analysis of the Signal Averaged Electrocardiogram in Patients with Ventricular Tachycardia and Coronary Artery Disease: Methodologic Validation and Clinical Relevance. JACC 1988; 12:150

4. Burg JP., Maximum Entropy Spectral Analysis, 37th Meeting of Society of Exploration Geophysicists. In: Modern Spectrum Analysis. Editor Childers DG., IEEE Press, New York (1978)

5. Friedlander B., System Identification Techniques for Adaptive Noise Cancelling, IEEE Transactions on Acoustics, Speech and Signal Processing. Vol. ASSP-30, No. 5 (1822)

6. Kalouptsidis N, Theodoridis S.: Fast Adaptive Least Squares Algorithms for Power Spectral Estimation, IEEE Transactions on Acaustics, Speech and Signal Processing, Vol. 63, No. 4 (1975)

7. Makhaul J. : Linear Prediction : a tutorial review, Proceedings of the JEEE, Vol 63, No 4 (1975)

8. Ibrahim M.K. : On line splitting in the optimum tapered Burg algorithm. JEEE Transactions on acoustics, speech, and signal processing, Vol 35, No 10 (1987)

## Anhang 2

```
Programm zur Berechnung der Filterkoeffizienten nach MEM
'..
'.
'
Integer Ord, PBest, Start, SegSize, Taper, Wik, Nord, Nu, W
Integer I, L, P, T
'
'Ord, PBest : maximale bzw. optimale Ordnung
'Start, SegSize : Startpunkt und Segmentlänge der Daten
'Taper, Wik, Nord, Nu : Variable zur Berechnung der Taperfkt
'W : Variable zur Dimensionierung benötigter Felder (W= 250)
'I, L, P, T : Schleifen-, Lauf- oder Hilfsindices
'
'
Real Nom, Den, Ena, Ene, Prod, Produc, Startaic, Relerr, Eno
Real G
'
'Nom, Den : Nenner und Zähler in der Koeffizientenberechnung
'Ena, Ene : Mittelwert und Varianz des Eingangssignals
'Prod, Produc : aktueller Wert bzw.Endwert des Filter-Output
'Startaic : Startwert des Filter-Output
'Relerr : Schranke für Abbruchkriterium
'Eno : Hilfsvariable
'G : Filterkoeffizient
'
'
Allocate Fdu(W), Fpe2(W), Ad(W), Help(W)
'
'Fdu : Daten (Übergabe aus dem Hauptprogramm als Signal(*))
'Fpe2 : relative Fehler (Abbruchkriterien)
'Ad   : Filterkoeffizienten
'Help : Hilfsfeld für Zwischen- oder Umspeicherungen
'
'
'Characters : AK$
'
'AK$ : automatische Suche der optimalen Ordnung (Ja/Nein)
'
'
'
```

13

```
' ******************** BEGINN  ********************
'
'
' ___  Umspeichern der Daten auf das Feld Fdu
'
MAT Fdu= Signal(Start:Start+SegSize-1)
'
' ___  Berechnung der maximalen Ordnung
'
Ord=INT(3*SQR(SegSize))
'
' ___  Berechnung von Mittelwert und Varianz
'
Ena=SUM(Fdu)/SegSize
'
MAT Fdu= Fdu-(Ena)
MAT Help= Fdu . Fdu
Eno=SUM(Help)/(SegSize-1)
Ene=SQR(Eno)
'
' ___  Berechnung des ersten Filteroutput
'
Startaic=(SegSize+1)/(SegSize-1)/Eno
'
' ___  Vorbelegungen einzelner Variablen
'
RESTORE 10
10 DATA 1,1,1,0,1,2
READ Produc, Prod, PBest, P, Wik, Nu
'
' ___r_r_r_r_r___  Rekursionssprungziel ___r_r_r_r_r___
100 P=P+1
    L=P+1
    Nom=0
    Den=0
'
' ___  Berechnung des Filterkoeffizienten
'
FOR I=L TO SegSize
'
'      ___  Berechnung der Taperfunktion
'
'Möglich : Taper der Ordnung : 0, 2, P
'Taper der P-ten Ordnung dämpft stärker
'als Taper 2. Ordnung
'
IF Taper=1 THEN Nu=P
IF Taper<>0 THEN

     (hier folgen weitere Programmschritte)

'
Nom=Nom ...
Den=Den ...
NEXT I
'
G=DROUND(2*Nom/Den,8)
'Ablage des neuen Koeffizienten in das Feld Fdu(*)
Fdu(P)=G
```

```
'
'
' ___ Berechnung des neuen Filteroutput
'
Produc=Produc*(1-G*G)
'
' ___ Levinson/Durbin-Rekursion :
' ─── Filterkoeffizienten-"Update"
'
IF P>1 THEN
   L=INT(P/2)
   FOR I=1 TO L

        (weitere Programmschritte)

        ...
   NEXT I
END IF
'
' ___ Kriterium für Abbruch der Rekursion
' ─── ... (weitere Programmfolge)
Relerr= ...

IF P>1 AND AK$='J' THEN
            ...




END IF
IF P=1 OR AK$='N' THEN
      PBest=P
      Prod=Produc
END IF
'
'Abbruch, wenn P die maximale Ordnung Ord erreicht hat
'
IF P<Ord THEN
'
'Berechnung der neuen Fehlerfilterfunktion :
' "Update der Vorwärts- und Rückwärtsfunktionen"
'
L=P+1
FOR I=SegSize TO L STEP -1


        ...

NEXT I
Ad(P)=G
GOTO 100
END IF
'
'Ende der Koeffizientenberechnung,
'   Koeffizienten sind im Feld Fdu(*) abgelegt
'
```

```
'  ___  Berechnung des Spektrums
'
'
'  ___  Aufbereiten für die Übergabe des Feldes zur FFT
'
MAT Help= Fdu/(-1)
REDIM Fdu(0:Ord)
MAT Fdu(0:Ord-1)= Help(1:Ord)
Fdu(0)=1
'
'
'  ___  Übergabe zur FFT
'XA, YA : Hilfsfelder der FFT
'
Fft_Size=512
'
CALL FFT(XA, YA, Fdu, Fft_Size)
'
Prod=2*Prod/Fft_Size
L= Fft_Size DIV 2
'
'  ___  Spektrum MEM(*)
'
FOR I=1 TO L
    MEM(I)=Prod/(XA(I)^2+YA(I)^2)
NEXT I
'
'  *** Ende ***
```

## Anhang 3

**Programm zur Berechnung der Filterkoeffizienten nach AFB**

```
'
'
'
Integer Ord, PBest, Start, SegSize, W
Integer I, J, K, K_10, Merk
'
'Ord, PBest : maximale bzw. optimale Ordnung
'Start, SegSize : Startpunkt und Segmentlänge der Daten
'W : Variable zur Dimensionierung benötigter Felder (W= 250)
'J : Laufindex über Schleife1 für vorliegendes Signal
'I : Laufindex über Ordnung
'K, K_10, Merk : Schleifen- und Hilfsindices
'
'
Real Ena, Ene, Ene_merk, Enep, Prod, Relerr
Real Ec1, Ec2, Lw, Ew, Egw, Eu, Egu, Lu
Real Sum, Sum_1, K1, K2
Real Lm_11, Lm_22, Lm_12, Lm_21, B_11, B_22, B_12, B_21, Ek
'
'Ena, Ene : Mittelwert und Varianz des Eingangssignals
'Ene_merk : Startwert des Filter-Output
'Enep, Prod : aktueller Wert bzw.Endwert des Filter-Output
'Relerr : Schranke für Abbruchkriterium
'Ec1, Ec2, Lw, Ew, Egw, Eu, Egu, Lu, Sum, Sum1, K1, K2 :
'    Hilfsvariablen zur Filter-Output resp. Varianzberechnung
'    der Vorwärts- und Rückwärts- Fehlerfilterfunktionen
'Lm_11, Lm_22, Lm_12, L_m21, B_11, B_22, B_12, B_21, Ek :
'    Hilfsvariable zur Berechnung der Fehlerfilterfunktionen
'
'
Allocate  Ad(W), Fd(W)
Allocate  Xa(W), Ya(W)
Allocate  Fpe(W), Fpe2(W), Neu(W)
Allocate  Help(W), Help1(W), Help2(W)
'
'Fdu : Daten (Übergabe aus dem Hauptprogramm als Signal(*))
'Ad  : Daten, in umgedrehter Reihenfolge (jeweils von 1 bis Ord !)
'Fd  : Filterkoeffizienten
'Xa, Ya : Vorwärts- resp. Rückwärts-Fehlerfilterfunktionen
'Fpe : Gesamt Fehlerfunktion
'Fpe2 : Fehlersumme: vorwärts und rückwärts, Hilfsfeld
'Neu : relativer Fehler (Abbruchkriterien)
'Help, Help1, Help2 :
'      Hilfsfelder für Zwischen- oder Umspeicherungen
'
'
'  ˙
'
```

17

```
' ******************* BEGINN   *******************
'
'
' ____ Umspeichern der Daten auf das Feld Fdu
'
MAT Fdu= Signal(Start:Start+SegSize-1)
'
' ____ Festlegung der maximalen Ordnung
'
Ord=50
If Ord>SegSize Then Ord=SegSize
'
' ____ Berechnung von Mittelwert und Varianz
'
Ena=SUM(Fdu)/SegSize
'
MAT Fdu= Fdu-(Ena)
MAT Help= Fdu . Fdu
Ene=SQR(SUM(Help)/(SegSize-1))
'
' ____ Umspeicherung und Vorbelegung einzelner Variablen
'
Ene_merk=Ene
Enep=Ene
'
RESTORE 10
10 DATA 1,1,0,0,0,0
READ Lm_11,Lm_22,Lm_12,Lm_21,Eu,Egu
'
'
'        ____r_r_r_r_r____ Rekursion ____r_r_r_r_r____
'
For J=1 To SegSize
'
'
' ____ Schritt 1,   Gesamt Fehlerfunktion
'
   For I=1 To Ord
       Fpe(I)=Ya(I)+Xa(I)*Egu
   Next I
'
'
' ____ Schritt 2,   Vorwärtsfehlerfunktion
'
Sum_1=0
K_10=Ord+1
For I=1 To Ord
     ...
Next I
Lw=Lm_22-Egu*Eu
Ew=Sum_1
Egw=Ew/Lw
Sum=0
For I=1 To Ord
     ...
Next I
```

```
'
'
'  ___  Schritt 3,    Rückwärtsfehlerfunktion
'
For I=1 To Ord
        . . .
Next I
K1=-(Sum+Fdu(J))/Ene
K_10=Ord+2
For I=1 To Ord
        . . .
NEXT I
Fpe2(1)=K1
K2=Fpe2(Ord+1)
For I=1 To Ord
      Ya(I)=Fpe2(I)-Fd(I)*K2
NEXT I
'
'
'  ___  Schritt 4,    Berechnung der Filterkoeffizienten
'                      und Update der Fehlerfilterfunktionen
'
Ec1=-Ene*K1
Ec2=-Ene*K2
Lm_11=    . . .
Lm_22=    . . .
K_10=Ord+1
For I=1 To Ord-1
        . . .
Next I
Ad(1)=Fdu(J)
Sum=0
Sum=Dot(Ad,Xa)
'
'
' Ermittlung der optimalen Ordnung
'
Relerr=0
If Ya(Ord)<>0 Then Relerr=1-Sqr(Xa(Ord)/Ya(Ord))
Neu(J)=0
If Relerr>0 Then . . .
        . . .
                          'Abbruchkriterium erfüllt
End If
'
' Ende Ermittlung der optimalen Ordnung
'Abbruch, wenn die maximale Ordnung Ord ermittelt ist
'anschließend Neustart der Rekursion mit dieser Ordnung
'
'
'
```

EP 0 407 647 B1

```
Lm_21=-Sum
Lm_12=Lm_21
Eu=-Lm_12
Lu=Lm_11
Egu=Eu/Lu
' Invertieren der Lm_nn`s
Ek=Lm_11*Lm_22-Lm_12*Lm_21
B_22=Lm_11/Ek
B_12=-Lm_12/Ek
B_21=-Lm_21/Ek
B_11=Lm_22/Ek

   '
   '
   '      ...
   '
   '
For I=1 To Ord
     ...
  Next I
'
'

'  ____ Ende der Rekursion
'

'

' Umspeichern der Koeffizienten
For I=1 to Ord-1
     Fpe2(I+1)=Fd(Ord-I)
Next I
Mat Fd(1:Ord)=Fpe2(1:Ord)
Fd(0)=1
'
'  ____ Ende der Koeffizientenberechnung,
'        Koeffizienten sind im Feld Fd(*) abgelegt
'        (Stabilitätstest für die Koeffizienten möglich)
'        Aufbereiten für die Übergabe des Feldes zur FFT
'
'

'  ____ Berechnung des Spektrums
'        Übergabe zur FFT
'
'XA, YA : Hilfsfelder der FFT
'
Fft_Size=512
'
CALL FFT(XA, YA, Fd, Fft_Size)
'
Prod=(Ene/SegSize)^2
L= Fft_Size DIV 2
'
'  ____ Spektrum AFB(*)
'
FOR I=1 TO L
    AFB(I)=Prod/(XA(I)^2+YA(I)^2)
NEXT I

    * Ende *
```

**Patentansprüche**

1. Einrichtung zur rechnergestützten selektiven Bewertung von physiologischen Meßsignalen, welche nach Vorverstärkung und Impedanzwandlung (1) über einen Hauptverstärker (2) einer auf einen festen Spannungspegel normierten Feinverstärkung unterworfen und analog/digital gewandelt als Eingabedaten in einen Speicher eingelesen und durch den Rechner (5) bewertet werden,

20

EP 0 407 647 B1

**dadurch gekennzeichnet,** daß die im Speicher abgelegten Daten durch den Rechner (5) einer Frequenzanalyse unterzogen werden, bei der
- aus den im Speicher abgelegten Eingabedaten zunächst in Abhängigkeit von der Art der Bewertung ein zu analysierender Signalabschnitt hinsichtlich seines Beginns und Endes festgelegt wird,
- ausgehend vom Beginn oder Ende dieses Signalabschnitts eine Mehrzahl von um festgelegte gleiche Zeitdifferenzen gegeneinander versetzte Zeitsegmente des Meßsignals herausgegriffen und einzeln einer Spektralanalyse mit autoregressiven Rechenmethoden unterzogen werden, bei denen zwecks Minimierung von Grundrauschen und zur Unterdrückung von Störungen durch Grundschwingungen die Differenzen aus zwei Modellrechnungen mit autoregressivem Algorithmus bei stark unterschiedlichen Ordnungszahlen zur Bestimmung der spektralen Leistungsdichte ermittelt werden,
- bezogen auf den möglichen Frequenzbereich von zu bewertenden Signalanteilen ein erster Teil der Zeitsegmente einer spektralen Bewertung der Leistungsamplituden unterzogen und zur Prüfung auf Störsignale eine ergänzende Vergleichsprüfung der übrigen Zeitsegmente durchgeführt wird, und bei der
- zur Ermittlung der spektralen Ausprägung der zu bewertenden Signalanteile das Flächenintegral der Spektren in dem bestimmten Frequenzbereich für die Spektren des ersten Teils sowie für die Spektren der übrigen Zeitsegmente berechnet wird zur Bestimmung eines Normalitätsfaktors (NF) als Quotient der aufsummierten spektralen Flächenintegrale von erstem und übrigem Teil der Zeitsegmente.

2. Einrichtung nach Anspruch 1 zur Bewertung von Elektrokardiogrammen (EKGs), dadurch gekennzeichnet, daß
- für die Bestimmung des zu analysierenden Signalabschnitts aus den im Speicher abgelegten Eingabedaten zunächst ein Raumvektor der EKG-Änderung bestimmt wird,
- Beginn und Ende des QRS-Komplexes durch Feststellen der Änderungsgeschwindigkeit des Raumvektors und Bestimmung der Faltungsmaxima dieser Geschwindigkeitsfunktion in einem bestimmten Zeitabschnitt festgelegt werden, und daß die Festlegung der Mehrzahl von gegeneinander versetzter Zeitsegmente ausgehend vom Beginn oder Ende des QRS-Komplexes erfolgt.

3. Einrichtung nach Anspruch 2 zur Erkennung und Bewertung von Spätpotentialen in EKGs, dadurch gekennzeichnet, daß
- bei der Bestimmung von Beginn und Ende des QRS-Komplexes die Änderungsgeschwindigkeit des EKG-Raumvektors zunächst gegenüber einem festgelegten Grenzwert von z. B. 20 mV/s abgeschätzt und sodann die Festlegung der Faltungsmaxima dieser Geschwindigkeitsfunktion in dem bestimmten Zeitabschnitt erfolgt,
- ausgehend vom Ende des QRS-Komplexes die Mehrzahl von um die festgelegten gleichen Zeitdifferenzen gegeneinander versetzten Zeitsegmente innerhalb der ST-Strecke des EKG herausgegriffen und einzeln einer Spektralanalyse mit der autoregressiven Rechenmethode unterzogen werden, bei der zwecks Minimierung des Grundrauschens und zur Unterdrückung von Störungen durch Grundschwingungen dies Differenzen aus zwei Modellrechnungen mit autoregressivem Algorithmus bei stark unterschiedlichen Ordnungszahlen zur Bestimmung der spektralen Leistungsdichte ermittelt werden, und daß
- die beiden Teile der Zeitsegmente in einem für Spätpotentiale charakteristischen Frequenzbereich gewählt werden.

4. Einrichtung nach Anspruch 3, **dadurch gekennzeichnet,** daß die Spektren im Frequenzbereich von 40 Hz bis 170 Hz, vorzugsweise im Bereich von 40 Hz bis 160 Hz untersucht werden, und daß bei der Bewertung der Leistungsamplituden die ergänzende Vergleichsprüfung der übrigen Zeitsegmente in Abhängigkeit davon durchgeführt wird, ob für den ersten Teil der Zeitsegmente ein festgelegter Wert der Leistungsamplituden überschritten wird.

5. Einrichtung nach Anspruch 4, **dadurch gekennzeichnet,** daß die Oberflächen-EKGs von drei vorzugsweise bipolaren, am Körper einer zu untersuchenden Person im wesentlichen rechtwinkliger Anordnung zu fixierenden Elektrodenpaaren (X, Y, Z) aufgenommenen orthogonalen EKGs als Signale für die Eingabedaten verwendet werden.

21

**6.** Einrichtung nach Anspruch 4, **dadurch gekennzeichnet,** daß die Signale eines intrakardial aufgenommenen EKGs als Eingabedaten verwendet werden.

**7.** Einrichtung nach Anspruch 2, **dadurch gekennzeichnet,** daß zur Erfassung von Potentialen des Vorhofs und des His' schen Bündels Zeitsegmente im Bereich der P-Welle bis zum Beginn des QRS-Komplexes untersucht werden.

**8.** Einrichtung nach Anspruch 2, **dadurch gekennzeichnet,** daß zur Erfassung von Abstoßungsreaktionen nach Herztransplantation Zeitsegmente der P-Welle, des QRS-Komplexes selbst und der ST-Strecke untersucht und die Spektrale Ausprägung für einen Frequenzbereich von 50 Hz bis 160 Hz bestimmt wird.

**9.** Einrichtung nach Anspruch 2, **dadurch gekennzeichnet,** daß 20 bis 60 Zeitsegmente mit Bezug auf das QRS-Ende oder den QRS-Beginn herausgegriffen werden, wobei die Zeitverschiebung zwischen den Segmenten, bezogen auf den Herzzyklus, jeweils 1 bis 5 ms, vorzugsweise 2 ms, beträgt.

**10.** Einrichtung nach Anspruch 4, **dadurch gekennzeichnet,** daß 20 bis 60 gleich lange Zeitsegmente innerhalb der ST-Strecke herausgegriffen werden, wobei das erste Segment eine festgelegte Zeitspanne nach dem Ende des QRS-Komplexes gestartet und die übrigen Zeitsegmente, bezogen auf den Herzzyklus, jeweils um 1 bis 5 ms, vorzugsweise 2 ms, jeweils früher beginnend gewählt werden.

**11.** Einrichtung nach Anspruch 1, **dadurch gekennzeichnet,** daß die Spektralanalyse der einzelnen Zeitsegmente mittels eines autoregressiven Algorithmus der Maximum-Entropie-Methode (MEM) erfolgt, bei dem das Leistungsdichtespektrum MEM (M,n) bestimmt ist durch die Beziehung

$$\mathrm{MEM(M,n)} = \sigma^2 \cdot \delta t / \left[ \sum_{m=1}^{M} a(M,m) \cdot \exp\{-2 \cdot \pi \cdot i \cdot (m-1) \cdot n \cdot \delta t\} \right]^2$$

mit

$\sigma^2$:         spektrale Leistungsdichte des weißen Rauschens,

N:         Anzahl der verfügbaren Daten, n = 1, ..., N;

M:         Modell-Ordnung, m = 1, ..., M;

$\delta t$:         Abtastrate

a(M,m):         Koeffizienten des Prädiktions-Fehlerfilters mit der Länge M und a(M,1) = 1,

und bei dem ausgehend von einer Funktion, welche die spektrale Leistungsdichte $\sigma^3$ von weißem Rauschen repräsentiert, die Bestimmung der Koeffizienten a(M,m) des Prädiktions-Fehlerfilters unter Vorgabe eines ersten Werts für die Modell-Ordnung M rekursiv mit Inkrementierung von m bis zur Erfüllung einer Abbruchbedingung erfolgt, die aus der stufenweisen Betrachtung von drei Kriterien festgelegt wird, mit Abbruch der Rekursion, falls

- zum einen für das Fehlerkriterium FPE (m) ~ (n÷m) / (n-m) gilt:

FPE(m) > FPE(m-1),

- zum zweiten, falls m = 3 $\cdot \sqrt{N}$ erfüllt ist und
- zum dritten, falls der Koeffizient a(M, m) $\geq$ 1 für m > 1 wird.

**12.** Einrichtung nach Anspruch 11, **dadurch gekennzeich**net, daß die Zeitsegmente 30 bis 70 ms, vorzugsweise 40 ms, lang sind.

**13.** Einrichtung nach Anspruch 10, **dadurch gekennzeichnet**, daß die Filterkoeffizienten durch eine Taperfunktion

T (m,n) = 6$\cdot$(m + 1)$\cdot$(N-n-m + 1)/[(N-n + 1)$\cdot$(N-n + 2)$\cdot$(N-n + 3)]

gewichtet werden zur Verminderung von Frequenzverschiebung und Aufsplittung von Frequenzgipfeln, wobei gilt

m = 1, ..., M; M: Modellordnung und

n = 1, ..., N; N: Anzahl der Daten.

**14.** Einrichtung nach Anspruch 1, **dadurch gekennzeichnet,** daß die Spektralanalyse der einzelnen Zeitsegmente mittels eines autoregressiven Algorithmus erfolgt, bei dem das Leistungsspektrum AFB (M,n) bestimmt ist durch die Beziehung

$$AFB(M,n) = \sigma^2 \delta t / \left[ \sum_{m=1}^{M} a(M,m) \cdot \exp\{-2 \cdot \pi \cdot i \cdot (m-1) \cdot n \cdot \delta t\} \right]^2$$

mit

$\sigma^2$:   Spektrale Leistungsdichte von weißem Rauschen,

N:   Anzahl der verfügbaren Daten, n = 1, ...n, N;

M:   Modell-Ordnung, m = 1, ..., M;

$\delta$t:   Abtastrate und

a(M,m):   Filterkoeffizienten,

und bei dem ausgehend von einer Funktion, welche die spektrale Leistungsdichte von weißem Rauschen repräsentiert, die Berechnung der Anzahl der Koeffizienten a(M,m) zur Minimierung der Vorwärts- und Rückwärts-Fehlerfilterfunktion unter Vorgabe einer festen oberen Schranke für die Modell-Ordnung M rekursiv mit Inkrementierung von m bis zur Erfüllung einer Abbruchbedingung erfolgt, die zunächst durch eine obere Schranke bei m = 50, aber maximal m = M festgelegt und durch den aktuellen Laufindex n der Rekursion, der aus dem Abbruchkriterium ermittelt wird, das durch die Abweichung des jeweils letzten Werts der Fehlerfilterfunktion von einem festgelegten Wert bestimmt ist und, falls der Abbruch der Rekursion dadurch nicht erreicht wird, durch das Kriterium m = $3 \cdot \sqrt{N}$ erzwungen wird.

**15.** Einrichtung nach Anspruch 11 oder 14, **dadurch gekennzeichnet,** daß die Zahl M der Modell-Ordnung bei der zweiten Modellrechnung zur Differenzspektrenberechnung zu M≦3 gewählt ist.

**16.** Einrichtung nach Anspruch 15, **dadurch gekennzeichnet,** daß die Länge der Zeitsegmente 20 bis 60 ms, vorzugsweise 25 ms, beträgt.

## Claims

**1.** Device for the computer-assisted selective evaluation of physiological measurement signals which, after preamplification and impedance transformation (1) by means of a main amplifier (2), are subjected to an incremental amplification normalised to a fixed voltage level and, in analog/digital converted form, are read into a memory as input data and evaluated by the computer (5), characterised in that the data filed in the memory are subjected by the computer (5) to a frequency analysis, in which

- from the input data filed in the memory a signal portion to be analysed is firstly specified with respect to its start and end as a function of the nature of the evaluation,

- proceeding from the start or end of this signal portion, a plurality of time segments of the measurement signal, which are mutually shifted by specified equal time differences, are picked out and are individually subjected to a spectral analysis using autoregressive computing methods, in which for the purpose of minimisation of background noises and for the suppression of disturbances due to fundamental oscillations, the differences of two model computations using an autoregressive algorithm with greatly differing order numbers are calculated for the determination of the spectral power density,

- related to the possible frequency range of signal components to be evaluated, a first part of the time segments is subjected to a spectral evaluation of the power amplitudes and, in order to test for disturbance signals, a supplementary comparative test of the remaining time segments is carried out, and in which

- to calculate the spectral identity of the signal components to be evaluated, the surface integral of the spectra is computed in the determined frequency range for the spectra of the first part as well as for the spectra of the remaining time segments, for the determination of a normality factor (NF) as quotient of the summed spectral surface integrals of the first and the remaining part of the time segments.

2. Device according to Claim 1 for the evaluation of electrocardiograms (ECGs), characterised in that
   - for the determination of the signal portion to be analysed firstly a space vector of the ECG change is determined from the input data filed in the memory,
   - start and end of the QRS complex are specified by establishing the rate of change of the space vector and determination of the folding maxima of this rate function in a determined time interval, and in that the specification of the plurality of mutually shifted time segments takes place proceeding from the start or end of the QRS complex.

3. Device according to Claim 2 for the recognition and evaluation of late potentials in ECGs, characterised in that
   - in the determination of start and end of the QRS complex the rate of change of the ECG vector is firstly estimated in relation to a specified limiting value of, for example, 20 mV/s, and then the specification of the folding maxima of this rate function in the determined time interval takes place,
   - proceeding from the end of the QRS complex, the plurality of time segments which are mutually shifted by the specified equal time differences are picked out within the ST section of the ECG and are individually subjected to a spectral analysis using the autoregressive computing method, in which for the purpose of minimisation of the background noise and for the suppression of disturbances due to fundamental oscillations, the differences of two model computations using an autoregressive algorithm with greatly differing order numbers are calculated for the determination of the spectral power density, and in that
   - the two parts of the time segments are selected in a frequency range which is characteristic for late potentials.

4. Device according to Claim 3, characterised in that the spectra are investigated in the frequency range of 40 Hz to 170 Hz, preferably in the range from 40 Hz to 160 Hz, and in that in the evaluation of the power amplitudes the supplementary comparative test of the remaining time segments is carried out in dependence upon whether for the first part of the time segments a specified value of the power amplitudes is exceeded.

5. Device according to Claim 4, characterised in that the surface ECGs of three preferably bipolar pairs of electrodes (X, Y, Z) to be fixed on the body of a person to be examined in a substantially rectangular arrangement [lacuna] recorded orthogonal ECGs are used as signals for the input data.

6. Device according to Claim 4, characterised in that the signals of an intracardially recorded ECG are used as input data.

7. Device according to Claim 2, characterised in that to determine potentials of the atrium and of the bundle of His, time segments are investigated in the range of the P wave up to the start of the QRS complex.

8. Device according to Claim 2, characterised in that to determine rejection reactions after a heart transplant, time segments of the P wave, of the QRS complex itself and of the ST section are investigated and the spectral identity is determined for a frequency range of 50 Hz to 160 Hz.

9. Device according to Claim 2, characterised in that 20 to 60 time segments are picked out with reference to the QRS end or the QRS start, the time shift between the segments being in each instance 1 to 5 ms, preferably 2 ms, related to the cardiac cycle.

10. Device according to Claim 4, characterised in that 20 to 60 equally long time segments are picked out within the ST section, the first segment being started a specified time interval after the end of the QRS complex and the remaining time segments being selected, related to the cardiac cycle, to start in each instance 1 to 5 ms, preferably 2 ms, earlier.

**11.** Device according to Claim 1, characterised in that the spectral analysis of the individual time segments takes place by means of an autoregressive algorithm of the maximum entropy method (MEM), in which algorithm the power density spectrum MEM (M, n) is determined by the relation

$$\text{MEM (M, n)} = \sigma^2 \cdot \delta t / [\sum_{m=1}^{M} a(M,m) \cdot \exp\{-2 \cdot \pi \cdot i \cdot (m-1) \cdot n \cdot \delta t\}]^2$$

with

$\sigma^2$:      spectral power density of the white noise,

N:      number of available data, n = 1, ..., N;

M:      model order, m = 1, ..., M;

$\delta t$:      scan rate

a(M,m):      coefficients of the prediction error filter with the length M and a(M,1) = 1,

and in which, proceeding from a function which represents the spectral power density $\sigma^2$ of white noise, the determination of the coefficients a(M,m) of the prediction error filter takes place with predetermination of a first value for the model order M recursively with incrementation of m up to the fulfilment of an interrupt condition, which is specified from the stepwise consideration of three criteria, with interruption of the recursion [sic], in the event that

- on the one hand, for the error criterion FPE(m) ~ (n÷m)/(n-m), the following is applicable:

    FPE(m) > FPE(m-1),

- secondly, in the event that m = 3 •$\sqrt{N}$ is satisfied and
- thirdly, in the event that the coefficient a(M, m) ≥ 1 for m > 1.

**12.** Device according to Claim 11, characterised in that the time segments have a length of 30 to 70 ms, preferably 40 ms.

**13.** Device according to Claim 10, characterised in that the filter coefficients are weighted by a taper function

T(m,n) = 6•(m + 1)•(N-n-m + 1)/[(N-n + 1)•(N-n + 2)•(N-n + 3)]

to reduce frequency shift and splitting of frequency peaks, the following being applicable:

m = 1, ..., M; M: model order and

n = 1, ..., N; N: number of data.

**14.** Device according to Claim 1, characterised in that the spectral analysis of the individual time segments takes place by means of an autoregressive algorithm, in which the power spectrum AFB(M,n) is determined by the relation

$$\text{AFB(M, n)} = \sigma^2 \delta t / [\sum_{m=1}^{M} a(M,m) \cdot \exp\{-2 \cdot \pi \cdot i \cdot (m-1) \cdot n \cdot \delta t\}]^2$$

with

$\sigma^2$:      spectral power density of white noise,

N:      number of available data, n = 1, ...n, N;

M:      model order, m = 1, ..., M;

$\delta t$:      scan rate and

a(M,m):      filter coefficients,

and in which, proceeding from a function which represents the spectral power density of white noise, the computation of the number of coefficients a(M,m) takes place, to minimize the forward and reverse error filter function, with predetermination of a fixed upper bound for the model order M recursively with

EP 0 407 647 B1

incrementation of m up to the satisfaction of an interrupt condition, which is specified firstly by an upper bound at m = 50, but as a maximum m = M, and is calculated by the current progressive index n of the recursion, which index is calculated from the interrupt criterion which has been determined by the deviation of the respectively last value of the error filter function from a specified value and, in the event that the interruption of the recursion is not reached thereby, is compelled by the criterion m = 3•√N.

15. Device according to Claim 11 or 14, characterised in that the number M of the model order is selected to be M≤3 in the second model computation for the computation of differential spectra.

16. Device according to Claim 15, characterised in that the length of the time segments is 20 to 60 ms, preferably 25 ms.

**Revendications**

1. Dispositif pour l'évaluation sélective, assistée par ordinateur, de signaux de mesure physiologiques qui, après une préamplification et une adaptation d'impédance (1), sont soumis, au moyen d'un amplificateur principal (2), à une amplification fine normalisée à un niveau de tension fixe, puis sont, après numérisation, enregistrés comme données d'entrée dans une mémoire et évalués par l'ordinateur (5), **caractérisé** en ce que les données déposées dans la mémoire sont soumises par l'ordinateur (5) à une analyse de fréquences selon laquelle :
   - à partir des données d'entrée déposées dans la mémoire, on détermine d'abord, en fonction du type d'évaluation, le début et la fin d'une partie de signal à analyser,
   - en partant du début ou de la fin de cette partie de signal, on prélève une pluralité de segments de temps du signal de mesure, mutuellement décalés de différences de temps identiques fixées, et on les soumet individuellement à une analyse spectrale avec des méthodes de calcul autorégressives, selon lesquelles, afin de minimiser le bruit de fond et d'éliminer les perturbations dues à des oscillations fondamentales, on détermine les différences de deux modèles de calcul à algorithme auto-régressif de numéros d'ordre nettement différents, en vue de déterminer la densité spectrale de puissance,
   - par rapport à la plage envisageable de fréquences des parties de signal à évaluer, on soumet une première partie des segments de temps à une évaluation spectrale des amplitudes de puissance et, afin de contrôler les signaux parasites, on réalise un contrôle comparatif complémentaire des autres segments de temps, et selon laquelle
   - afin de déterminer l'empreinte spectrale des parties de signal à évaluer, on calcule l'intégrale de surface des spectres dans la plage déterminée de fréquences pour les spectres de la première partie ainsi que pour les spectres des autres segments de temps, afin de déterminer un facteur de normalité (NF) comme quotient des intégrales de surface spectrale totalisées de la première partie et de la partie restante des segments de temps.

2. Dispositif selon la revendication 1 pour l'évaluation d'électrocardiogrammes (E.C.G.), **caractérisé** en ce que
   - pour la détermination de la partie de signal à analyser, on détermine d'abord, à partir des données d'entrée déposées dans la mémoire, un vecteur spatial de la modification d'E.C.G.,
   - on détermine le début et la fin du complexe QRS en établissant la vitesse de modification du vecteur spatial et en déterminant les maxima de convolution de cette fonction de vitesse dans un intervalle de temps donné,
   et en ce que la détermination de la pluralité de segments de temps mutuellement décalés s'effectue en partant du début ou de la fin du complexe QRS.

3. Dispositif selon la revendication 2 pour l'identification et l'évaluation de potentiels retardés dans des électrocardiogrammes, **caractérisé** en ce que
   - lors de la détermination du début et de la fin du complexe QRS, on évalue d'abord la vitesse de modification du vecteur spatial d'E.C.G. par rapport à une valeur limite définie, égale par exemple à 20 mV/s, puis on effectue la détermination des maxima de convolution de cette fonction de vitesse dans l'intervalle de temps déterminé,
   - en partant de la fin du complexe QRS, on prélève la pluralité de segments, mutuellement décalés de différences de temps identiques fixées, à l'intérieur du segment ST de l'E.C.G., et on les soumet individuellement à une analyse spectrale avec la méthode de calcul auto-régressive,

26

selon laquelle, afin de minimiser le bruit de fond et d'éliminer les perturbations dues à des oscillations fondamentales, on détermine les différences de deux modèles de calcul à algorithme auto-régressif de numéros d'ordre nettement différents, en vue de déterminer la densité spectrale de puissance,

et en ce que
- les deux parties des segments de temps sont choisies dans une plage de fréquences caractéristique de potentiels retardés.

4. Dispositif selon la revendication 3, **caractérisé** en ce que les spectres sont analysés dans la plage de fréquences de 40 Hz à 170 Hz, de préférence dans la plage de 40 Hz à 160 Hz, et en ce que, lors de l'évaluation des amplitudes de puissance, le contrôle comparateur complémentaire des autres segments de temps est effectué en fonction du dépassement d'une valeur définie des amplitudes de puissance pour la première partie des segments de temps.

5. Dispositif selon la revendication 4, **caractérisé** en ce qu'on utilise comme signaux pour les données d'entrée les électrocardiogrammes de surface orthogonaux enregistrés par trois paires d'électrodes de préférence bipolaires (X, Y, Z), à fixer en un agencement sensiblement orthogonal sur le corps d'une personne à analyser.

6. Dispositif selon la revendication 4, **caractérisé** en ce qu'on utilise comme données d'entrée les signaux d'un électrocardiogramme enregistré de façon intracardiaque.

7. Dispositif selon la revendication 2, **caractérisé** en ce qu'afin de détecter des potentiels d'oreillette et du faisceau de His, on analyse des segments de temps dans la région de l'onde P jusqu'au début du complexe QRS.

8. Dispositif selon la revendication 2, **caractérisé** en ce qu'afin de détecter des réactions de rejet à la suite d'une transplantation cardiaque, on analyse des segments de temps de l'onde P, du complexe QRS proprement dit et du segment ST, et on détermine l'empreinte spectrale pour la plage de fréquences de 50 Hz à 160 Hz.

9. Dispositif selon la revendication 2, **caractérisé** en ce qu'on prélève 20 à 60 segments de temps par rapport à la fin ou au début du complexe QRS, le décalage de temps entre les segments, rapporté au cycle cardiaque, étant chaque fois de 1 à 5 ms, de préférence de 2 ms.

10. Dispositif selon la revendication 4, **caractérisé** en ce que on prélève 20 à 60 segments de temps de même longueur dans le segment ST, le premier segment de temps commençant au bout d'un intervalle de temps fixé suivant la fin du complexe QRS, et les autres segments de temps, rapportés au cycle cardiaque, étant chaque fois choisis en commençant plus tôt de 1 à 5 ms, de préférence de 2 ms.

11. Dispositif selon la revendication 1, **caractérisé** en ce que l'analyse spectrale des différents segments de temps s'effectue au moyen d'un algorithme auto-régressif de la méthode d'entropie maximale (MEM), selon laquelle le spectre de densité de puissance MEM (M,n) est déterminé par la relation :

$$\text{MEM } (M,n) = \sigma^2 \cdot dt \; / \; [\sum_{m=1}^{M} a(M,m) \cdot \exp\{-2.\pi.i.(m-1).n.\delta t\} \;]^2$$

où :
$\sigma^2$ =  densité spectrale de puissance du bruit blanc
N =  nombre de données disponibles, n = 1, ..., N
M =  numéro d'ordre du modèle, m = 1, ..., M
$\delta t$ =  vitesse de balayage
a(M,m) =  coefficients du filtre d'erreur de prédiction, de longueur M avec a(M,1) = 1,
et selon laquelle, en partant d'une fonction représentant la densité spectrale de puissance $\sigma^2$ du bruit blanc, le coefficient a(M,M) du filtre d'erreur de prédiction s'obtient, en allouant une première valeur pour le modèle d'ordre M, de manière récurrente avec incrémentation de m jusqu'à ce que soit remplie une condition d'interruption qui est déterminée par l'observation progressive de trois critères, la récurrence étant interrompue si
- premièrement, le critère d'erreur FPE(m) $\approx$ (n + m) / (n-m) satisfait à la relation :

FPE(m) > FPE(m-1),

- deuxièmement, la condition m = 3 . $\sqrt{N}$ est remplie,
- troisièmement, le coefficient a(M,m) est $\geq$ 1 pour m > 1.

**12.** Dispositif selon la revendication 11, **caractérisé** en ce que la longueur des segments de temps est de 30 à 70 ms, de préférence de 40 ms.

**13.** Dispositif selon la revendication 10, **caractérisé** en ce qu'afin de réduire le décalage de fréquence et de morceler les pointes de fréquence, les coefficients de filtrage sont pondérés par une fonction d'atténuation :

T(m,n) = 6 . (m + 1) . (N-n-m + 1) / [(N-n + 1) . (N-n + 2) . (N-n + 3)]

où :
m = 1, ..., M ; M = numéro d'ordre du modèle, et
n = 1, ..., N ; N = nombre de données.

**14.** Dispositif selon la revendication 1, **caractérisé** en ce que l'analyse spectrale des différents segments de temps s'effectue au moyen d'un algorithme auto-régressif, selon lequel le spectre de puissance AFB(M,n) est déterminé par la relation :

$$\text{AFB(M,n)} = \sigma^2 \ . \ \text{dt} \ / \ [\sum_{m=1}^{M} a(M,m) \ . \ \exp\{-2.\pi.i.(m-1).n.\delta t\} \ ]^2$$

où :
$\sigma^2$ = densité spectrale de puissance du bruit blanc
N = nombre de données disponibles, n = 1, ..., N
M = numéro d'ordre du modèle, m = 1, ..., M
$\delta t$ = vitesse de balayage, et
a(M,m) = coefficients de filtrage,
et selon lequel, en partant d'une fonction représentant la densité spectrale de puissance du bruit blanc, le calcul du nombre de coefficients a(M,m) pour minimiser la fonction de filtrage d'erreur avant et arrière s'effectue, en allouant une barrière supérieure fixe pour le modèle d'ordre M, de manière récurrente avec incrémentation de m jusqu'à ce que soit remplie une condition d'interruption, qui est initialement définie par une barrière supérieure pour m = 50, avec toute-fois au plus m = M, et qui est déterminée à partir du critère d'interruption, qui est lui-même défini par l'écart de la dernière valeur respective de la fonction de filtrage d'erreur par rapport à une valeur fixée et qui, si l'interruption de la récurrence n est pas ainsi obtenue, est forcé par le critère m = 3 . $\sqrt{N}$.

**15.** Dispositif selon la revendication 11 ou 14, **caractérisé** en ce que le numéro d'ordre M du modèle est choisi $\leq$ 3 lors du second calcul de modèle pour le calcul des spectres différentiels.

**16.** Dispositif selon la revendication 15, **caractérisé** en ce que la longueur des segments de temps est de 20 à 60 ms, de préférence de 25 ms.

# SYSTEMAUFBAU

Fig. 1

## Auswertung: Flowchart

Einlesen der EKG-Werte
in das Computermemory

Graphische Darstellung in der
Zeitdomain (Amplitude vs. Zeit)

Bestimmung der QRS-Grenzen
über Autokorrelation

**Spektrotemporales Mapping**

For Segment = 1 to 30 (38)

| MEM | AFB |
|---|---|
| 1. Kalkulation mit niedriger Ordnung | 1. Kalkulation mit niedriger Ordnung |
| 2. Kalkulation mit hoher Ordnung | 2. Kalkulation mit hoher Ordnung |
| "Tapering" | |
| Differenz 2. - 1. | Differenz 2. - 1. |

Powerspektrum

Next Segment

3-dimensionaler Plot der Differenzspektren

Berechnung des Normalitätsfaktors

Fig. 2

Fig. 3

EP 0 407 647 B1

Fig. 4

Patient nach Herzinfarkt mit Spätpotentialen

Fig. 5

Herzgesunder ohne Spätpotentiale

Fig. 6
Signal mit Störeinflüssen ohne Spätpotentiale

## Fig. 7: FOURIER ANALYSE DER ST-STRECKE

Patient mit Spätpotentiale

QRS-Ende
−20

20 dB

+58

0    Frequenz ➡    150    Hz    300    0    CC    1

Herzgesunder ohne Spätpotentiale

QRS-Ende
−20

20 dB

+58

0    Frequenz ➡    150    Hz    300    0    CC    1

Signal mit Störeinflüssen

QRS-Ende
−20

20 dB

+58

0    Frequenz ➡    150    Hz    300    0    CC    1